(19) Europäisches Patentamt
European Patent Office
Office européen des brevets

(11) EP 1 808 172 A2

(12) EUROPEAN PATENT APPLICATION

(43) Date of publication:
**18.07.2007 Bulletin 2007/29**

(51) Int Cl.:
***A61K 31/352*** *(2006.01)*

(21) Application number: **07009055.0**

(22) Date of filing: **06.03.2003**

(84) Designated Contracting States:
**AT BE BG CH CY CZ DE DK EE ES FI FR GB GR HU IE IT LI LU MC NL PT RO SE SI SK TR**

(30) Priority: **06.03.2002 US 362420 P**
**22.04.2002 US 374417 P**

(62) Document number(s) of the earlier application(s) in accordance with Art. 76 EPC:
**03713959.9 / 1 487 434**

(71) Applicant: **The Medical Research and Education Trust**
**San Diego, CA 92101 (US)**

(72) Inventor: **Chen, Sophie**
**Millwood, NY 10546 (US)**

(74) Representative: **Leathley, Anna Elisabeth**
**Frank B. Dehn & Co.**
**St Bride's House**
**10 Salisbury Square**
**London EC4Y 8JD (GB)**

Remarks:
This application was filed on 04 - 05 - 2007 as a divisional application to the application mentioned under INID code 62.

# (54) Botanical extract compositions and methods of use

(57) A composition having phytoestrogenic and anti-cancer activity is described. The composition comprises wogonin, isoliquiritigenin, coumestrol, their pharmaceutically acceptable salts or esters, their selectively substituted analogs, or combinations thereof. The compositions may also include an anti-cancer agent and/or an immune stimulant. A method for treating or preventing cancer or an estrogen related disorder includes administering a therapeutically effective amount of the compositions is described. The compositions are particularly useful in the treatment of hormone-related cancers

FIG. 1

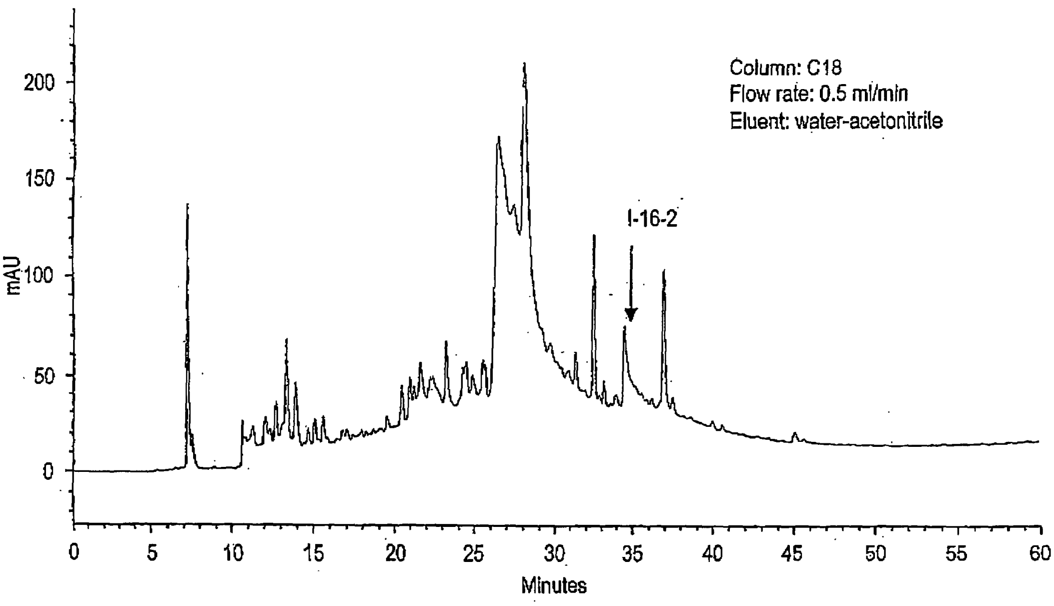




Printed by Jouve, 75001 PARIS (FR)

**Description**

TECHNICAL FIELD

This application related to botanical extract compositions and methods of treating humans, particularly methods of treating cancer and estrogen-related disorders.

BACKGROUND

Botanical extracts Phytoestrogens, such as the isoflavones contained in soy products, are believed to have therapeutic potential in disease treatment and prevention. In particular, phytoestrogens are believed to be useful in the treatment of estrogen-related disorders such as, for example, osteoporosis, the symptoms of menopause, and hormone-related cancers.

It has been reported that endogenous and exogenous hormones play a role in the development of hormone-related cancers, such as breast cancer, colon cancer, lung cancer, endometrial cancer ovarian cancer, prostate cancer, bladder cancer, testicular cancer, thyroid cancer, and bone cancer (see, for example, Henderson et al, "Hormonal carcinogenesis", Carcinogenesis (2000), 21(3): 427-433). Epidemiological studies have shown that consumption of a diet with high content of phytoestrogens such as those found in soy products was associated with a lower incidence of hormonal related cancers (H. Wiseman, "The therapeutic potential of phytoestragens", Expert. Opin. Investig. Drugs (2000), 9(8):1829-40).

Prostate carcinoma, a hormone-related cancer, is a major health problem among men in North America and Europe (S. H. Landis et al., "Cancer Statistics, 1998", CA Cancer J. Clin. (1998) 48: 6-29). Chronic enlargement of the prostate in combination with elevated prostate specific antigen (PSA) can often lead to prostate carcinoma. Every year 160,000 new cases and 39,000 deaths from the disease occur in the United States (Landis). Breast cancer, another hormone-related cancer, is also a major health problem. New invasive incidences of breast carcinoma are projected to be 192,200, with 40,200 projected deaths in 2001 according to the American Cancer Society (National Alliance of Breast Cancer Organizations News, 15(1): 2, January, 2001). Early detection and early intervention are often the key solutions to treating these diseases. Although chemotherapy is often the choice for advanced-stage breast cancer patients, for example, it is not effective for the advanced-stage prostate cancer patients. Conventional treatment methods include surgery, radiation, hormone therapy, and chemotherapy. There is a need for alternative therapeutic agents that can augment or replace existing therapies.

While the existing therapeutic agents are well-suited for their intended purpose, there remains a need for other herbal remedies for the treatment of estrogen-related disorders, including hormone-related cancers, in addition to remedies for non-hormone-related cancers.

BRIEF SUMMARY

Other embodiments, including compositions useful for treating cancer, are described in detail below.

BRIEF DESCRIPTION OF THE DRAWINGS

Figure 1 shows a high performance liquid chromatogram (measured at 254 nanometers) of a multi-component botanical extract composition containing extracts of Panax psendo-ginseng Wall, Isatis Indigotica Fort, Ganoderma lucidium Karst, Dendrathema morifolium Tzvel, Glycyrrhiza glabra L, Sculletaria bailcalensis Georgi, Rabdosia rubescens, and Serenoa repens; an arrow indicates the position of wogonin (designated "I-16-2") in the elution profile.

Figure 2 shows $^{13}C$ NMR spectra of wogonin separated from a multi-component botanical extract compositions as in Figure 1; (a) separate (DEPT) spectra for $-CH_3$, $-CH_2$ and -CH groups; (b) total $^{13}C$ NMR spectrum.

Figure 3 is a mass spectrum of wogonin separated from a multi-component botanical extract as in Figure 1, with a purity of greater than 95%.

Figure 4 is a high performance liquid chromatogram of isoliquiritigenin isolated from *Glycyrrhiza uralensis.*

Figure 5 is an absorption spectra associated with the isoliquiritigenin peak in the chromatogram of Figure 4.

Figure 6 shows $^{13}C$ NMR spectra of isoliquiritigenin separated from *Glycyrrhiza uralensis;* (a) separate (DEPT) spectra for $-CH_3$, $-CH_2$ and -CH groups; (b) total $^{13}C$ NMR spectrum.

Figure 7 is a mass spectrum of isoliquiritigenin separated from *Glycyrrhiza uralensi.*, with a purity shown to be higher than. 95%.

Figure 8 is a plot of cell viability of LNCaP and DU-145 prostate cancer cells as a function of wogonin concentration.

Figure 9 is a plot of cell viability of DU-145 and LNCaP prostate cancer cells, and MCF-7 breast cancer cells, as a function of isoliquiritigenin concentration.

Figure 10 displays DNA histograms showing the effect on LNCaP cell cycle in the absence (A) and presence (B) of wogonin at 20 micrograms/milliliter.

Figure 11 shows changes in the LNCaP cell cycle induced by wogonin and isoliquiritigenin.

Figure 12 shows changes in the DU-145 cell cycle induced by wogonin and isoliquiritigenin.

Figure 13 is a plot showing the potency of wogonin and isoliquiritigenin as ER-alpha-Luc reporter gene activation.

Figure 14 is a plot showing the potency of wogonin and isoliquiritigenin as ER-beta-Luc reporter gene activation.

Figure 15 is a plot of COX-2 inhibition as a function of isoliquiritigenin concentration.

Figure 16 is a plot of cell viability of PTX 10 ovarian cancer cells (resistant to taxol) in the presence of increasing concentrations of wogonin.

Figure 17 is a plot of cell viability of PTX 10 ovarian cancer cells in the presence of increasing concentrations of isoliquiritigenin

DETAILED DESCRIPTION OF THE PREFERRED EMBODIMENT

Disclosed herein are compositions and methods for treating cancer and estrogen-related disorders in a human un need of such treatment. As used herein, a human in need of cancer treatment may be a human diagnosed with cancer, or a human wanting to prevent or delay the onset of cancer, for example, a human with a family history of cancer. The cancer may optionally be a hormone-related cancer such as, for example, prostate cancer, breast cancer, endometrial cancer, colon cancer, lung cancer, bladder cancer, testicular cancer, ovarian cancer, thyroid cancer, or bone cancer. As used herein, a human in need of treatment for an estrogen-related disorder may be a human, diagnosed with an estrogen-related disorder such as, for example, osteoporosis or the symptoms of menopause, or a human wanting to prevent or delay the onset of an estrogen-related disorder. The method comprises administering a therapeutically effective amount of a composition comprising a phytoestrogen, such as, for example, wogonin, isoliquiritigenin, coumestrol, their pharmaceutically acceptable salts or esters, their selectively substituted analogs, and combinations comprising one or more of the foregoing compounds. As used herein, a phytoestrogen is a plant-derived compound or its metabolite that can mimic the action or modulate the binding, metabolism, or production af endogenous estrogens in the body.

As stated previously, herbal remedies have been used in the treatment of cancer. *Scutellaria baicalensis,* for example, is a source of wogonin (Y. Y. Zhang et al., "Comparative study of Scutellaria planipes and Scutellaria baicalensis", Biomed. Chromatogra, (1998), 12: 31-3), and *Glycyrrhiza uralensis* and *Glycyrrhiza glabra* are sources of isoliquiritigenin (H. Hayashi H. et al., "Seasonal variation of glycyrrhizain and isoliquiritgenin glycosides in the root of glycyrrhiza glabra L", Biol. Pharm. Bull. (1998) 21: 987-9).

Coumestrol is a phytoestrogen found in alfalfa and red clover that is known to exhibit phytoestrogenic activity (see, for example, U.S. Patent Application Publication No. 20010044431 A1 to Rodriguez).

Wogonin has been reported to be a strong anti-inflammation agent due to its inhibitory activity against cyclooxygenase 2 (COX-2) directly and against gene expression of inducible COX-2 and nitric oxide synthase (see, for example, Y. S. Chi et al., "Effect of wogonin, a plant flavone from Scutellaria radix, on the expression of cyclooxygenase-2 and the induction of inducible nitric oxide synthase , and the induction of inducible nitric oxide synthase in inhibitors and lipopolysaccharide-treated RAW 264.7 cells", Biochem. Pharmacol. (2001), 61 (11): 1417-27). However, the present inventor is aware of no reports of wogonin exhibiting estrogenic activity.

Isoliquiritigenin has been reported to possess estrogen-like activity (see, for example, S. Tamir "Estrogen-like

activity of glabrene and other constituents isolated from licorice root", J. Steroid Biochem. Mol. Biol. (2001), 78(3): 291-8). However, the present inventor is aware of no report that isoliquiritigenin is an inhibitor for COX-2 activity and thus is beneficial to treat cancer.

Recent studies have revealed the importance of COX-2 inhibitors as cancer therapeutic agents (see, for example, A. Kirschenbaum et al, "The role of cyclocxygenase-2 in prostate cancer" Urology (2001), 58(2 suppl. 1): 127-131; and E. T. Hawk et al., "COX-2 in Cancer-A Player That's Defining the Rules". J. Natl. Cancer Inst. (2002), 94(8): 545-546). The inhibitor blocks the angiogenesis of cancer and reduced the cancer metastasis (see, for example, E. Fosslien "Review: Molecular pathology of eyclooxygenas-2 in cancer-induced angiogenesis", Ann. Clin. Lab Sci. (2001), 31(4): 325-348). -

The present work demonstrates potent activity of wogonin and isoliquiritigenin to activate estrogen receptor-alpha and beta and trigger biochemical reactions in cancer cells. The COX-2 inhibitory activity of isoliquiritigenin is also demonstrated. Suppressive effects of both compounds on cancer cell proliferation are also demonstrated. The cytotoxicity of wogonin and isoliquiritigenin toward cancer cells may either be dependent or independent of estrogen receptors.

The phytoestrogen may comprise wogonin. As used herein, the term wogonin encompasses CAS Reg. No. 632-85-9, also known as 5,7-dihydroxy-8-methoxy-flavone, and its pharmaceutically acceptable salts or esters, its selectively substituted analogs, an extract from a plant of the *Scutellaria* family, or a combination comprising one or more of the foregoing compounds.

An ester of wogonin is preferably a glycoside of wogonin. There is no particular limit on the monosaccharide or polysaccharide used to form the glycoside of wogonin. Suitable monosaccharides sugars include, for example, glucose, glucuronic acid, mannose, fructose, galactose, xylose, rutinose, rhamnose, and the like, and combinations comprising one or more of the foregoing monosaccharides. Suitable polysaccharides include, for example, dimers, trimers, oligomers, and polymers formed from one or more of the above monosaccharides.

Wogonin analogs include, for example, genistein, biochanin, prunetin, scutellarein, daidzin, luteolin, apigenin, acacetin, 3,6,4-trihydoxylflavone, 7,3-dihydroxy-4,1-dimethoxy-isoflavone, 3R-2',3'-dihydoxy-7,4-dimethoxy-isoflavone, or the like, or a combination comprising one or more of the foregoing wogonin analogs.

Wogonin can also be in the form of an extract from a plant of the *Scutellaria* family such as, for example *Scutellaria baicalensis,* its pharmaceutically acceptable salts or esters, its selectively substituted analogs, or a combination comprising one or more of the foregoing compounds.

The wogonin can comprise a selectively substituted analog of wogonin having the formula

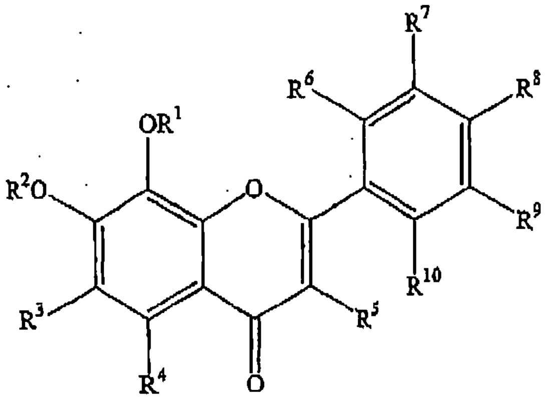

wherein $R^1$ is $C_1$-$C_6$ alkyl (preferably methyl); $R^2$ is hydrogen, $C_1$-$C_6$ alkyl, or $C_2$-$C_6$ acyl (preferably hydrogen); and $R^3$-$R^{10}$ are independently hydrogen, hydroxy, $C_1$-$C_6$ alkyl, $C_1$-$C_6$ alkoxy, or $C_2$-$C_6$ acyl (preferably hydrogen or hydroxy, more preferably hydrogen), with the proviso that at least four of $R^3$-$R^{10}$ are hydrogen. In a preferred embodiment, $R^1$ is methyl, $R^2$ is hydrogen, and $R^3$-$R^{10}$ are independently hydrogen, methyl, ethyl, methoxy, ethoxy, acetyl, or propionyl, with the proviso that at least five of $R^3$-$R^{10}$ are hydrogen.

Methods for synthesizing or isolating wogonin, its pharmaceutically acceptable salts or esters, its selectively substituted analogs, are known in the art. See, for example, International Patent Application No. WO01051482 A1 to Wallace et al; P. Rivaille et al., C. R. Acad. Sci., ,Paris, Ser. C (1969), 268(2): 2213-16; M. -C. Lin et al., J. Chromatogr, A (1999), 830(2): 387-395; Y. C. Chen et al, Biochem. Pharmacol. (2001), 61(11): 1417-1427.

When wogonin is present, the wogonin comprises greater than or equal to 0.5 weight percent, more preferably greater than or equal to about 1 weight percent, still more preferably greater than or equal to about 2 weight percent, even more preferably greater than or equal to about 5 weight percent, even more preferably greater than or equal to about 10 weight percent, still more preferably greater than, or equal to about 20 weight percent of the total weight of the

composition. Compositions containing as much as 50 weight percent, or even as much as 100 weight percent of wogonin, are contemplated.

The phytoestrogen may comprise isoliquiritigenin. As used herein, isoliquiritigenin refers to CAS Reg. No. 961-29-5; also known as 2',4,4'-trihydroxychalcone, a pharmaceutically acceptable salt or ester of isoliquiritigenin, a selectively substituted analog of isoliquiritigenin, an extract of *Glycyrrhiza uralensis* or *Glycyrrhiza glabra,* or a combination composing one or more of the foregoing compounds.

An ester of isoliquiritigenin is preferably a glycoside of isoliquiritigenin. There is no particular limit on the monosacharide or polysaccharide used to form the glycoside of isoliquiritigenin. Suitable monosaccharides sugars include, for example, glucose, glucuronic acid, mannose, fructose, galactose, xylose, rutinose, rhamnose, and the like, and combinations comprising one or more of the foregoing monosaccharides. Suitable polysaccharides include, for example, dimers, trimers, oligomers, and polymers fonned from one or more of the above monosaccharides.

An isoliquiritigenin analog includes, for example, phloretin, 4,2,4'-trihydroxychalcone, or the like, or a combination comprising one or more of the foregoing isoliquiritigenin analogs.

An extract of *Glycyrrhiza uralensis* or *Glycyrrhiza glabra* is a source of isoliquiritigenin, a pharmaceutically acceptable salt or ester of isoliquiritigenin, a selectively substituted analog of isoliquiritigenin, or a combination comprising one or more of the foregoing compounds.

A selectively substituted analog of isoliquiritigenin has the formula

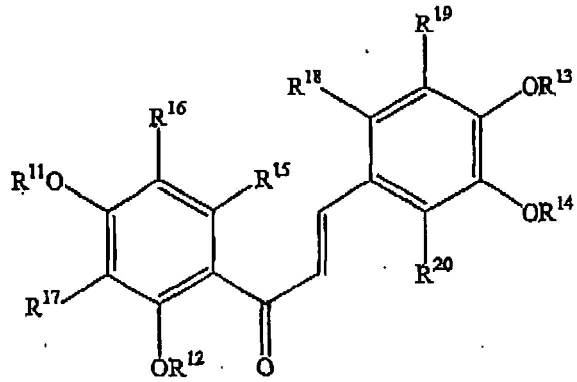

wherein $R^{11}$-$R^{14}$ are independently hydrogen or $C_1$-$C_6$ alkyl (preferably hydrogen); and $R^{15}$-$R^{20}$ are independently hydrogen, $C_1$-$C_6$ alkyl, $C_1$-$C_6$ alkoxy, or $C_2$-$C_6$ acyl (preferably hydrogen), with the proviso that at least three of $R^{15}$-$R^{20}$ are hydrogen. In a preferred embodiment, $R^{11}$-$R^{14}$ are hydrogen and $R^{15}$-$R^{20}$ are independently hydrogen, methyl, ethyl, methoxy, ethoxy, acetyl, or propionyl, with the proviso that at least four of $R^{15}$-$R^{20}$ are hydrogen.

Methods for synthesizing or isolating isoliquiritigenin, its pharmaceutically acceptable salts or esters, its selectively substituted analogs, are known in the art. See, for example, S. K. Srivastava et al., Indian J. Chem., Sect. B (1981), 20B(4): 347-8; and F. A. Macias et al., Phytochemistry (1998), 50(1): 35-46.

When itsoliquiritigenin is present, the isoliquiritigenin comprises greater than or equal to 0.5 weight percent, more preferably greater than or equal to about 1 weight percent, still more preferably greater than or equal to about 2 weight percent, even more preferably greater than or equal to about 5 weight percent, even more preferably greater than or equal to about 10 weight percent, still more preferably greater than or equal to about 20 weight percent of the total weight of the composition. Compositions containing as much as 50 weight percent, or even as much as 100 weight percent of isoliquiritigenin contemplated.

The phytoestrogen may comprise coumestrol. As used herein, coumestrol refers to CAS Reg. No. 479-13-0, also known as 3,9-dlydroxy-6H-bmzofaro[3.2-c][1]benzopyran 5-one, a pharmaceutically acceptable salt or ester of coumestrol, a selectively substituted analog of coumestrol, an extract of *Taraxacum mongoliocum,* alfalfa sprout (*Medicago sativa*), broccoli (*Brassica oleracea*)*, Eclipta prostrata,* or a combination comprising one or more of the foregoing compounds.

An ester of coumestrol is preferably a glycoside of coumestrol. There is no particular limit on the monosaccharide or polysaccharide used to form the glycoside of coumestrol. Suitable monosaccharides sugars include, for example, glucose, glucuronic acid, mannose, fructose, galactose, xylose, rutinose, rhamnose, and the like, and combinations comprising at least one of the foregoing monosaccharides. Suitable polysaccharides include, for example, dimers, trimers, oligomers, and polymers formed from one or more of the above monosaccharides.

A coumestrol analog includes, for example, 4-ethyl-7-hydroxy-3-(p-methoxyphenyl)-2H-1-benzopyran-2-one (wedelolactone), and the like.

The coumestrol may comprise an extract of *Taraxacum mongolicum,* alfafa sprout *(Medicago sativa),* broccoli *(Brassica oleracea), Ecltpta prostrata,* or the like as a source of coumestrol, a pharmaceutically acceptable salt or ester of coumestrol, a selectively substituted analog of coumestrol, or a combination comprising one or more of the foregoing compounds.

A selectively substituted analog of coumestrol has the formula

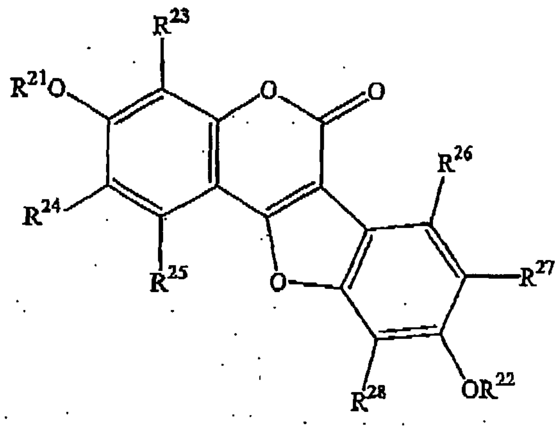

wherein $R^{21}$ and $R^{22}$ are independently hydrogen or $C_1$-$C_6$ alkyl (preferably hydrogen); and $R^{23}$-$R^{28}$ are independently hydrogen, $C_1$-$C_6$ alkyl, $C_1$-$C_6$ alkoxy, or $C_2$-$C_6$ acyl (preferably hydrogen), with the proviso that at least three of $R^{23}$-$R^{28}$ are hydrogen. In a preferred embodiment, $R^{21}$ and $R^{22}$ are hydrogen; and $R^{23}$-$R^{28}$ are independently hydrogen, methyl, ethyl, methoxy, ethoxy, acetyl, or propionyl, with the proviso that at least four of $R^{23}$-$R^{28}$ are hydrogen.

Methods for synthesizing or isolating coumestrol, its pharmaceutically acceptable salts or esters, its selectively substituted analogs, are known in the art. See, for example, P. M. Dewick et al., J. Chem. Soc. D (1969), 9: 466-7; T. Kappe et al., Z Naturforsch.; Teil B (1974), 29(3-4); 292-3; R Laschober et al., Synthesis (1990), 5: 387-8; F. A. Macias et al., Phytochemistry (1998), 50(1): 35-46; , K. Hiroya et al. Perkin 1 (2000), 24: 4339-4346; G. A. Kraus et al., Journal of Organic Chemistry (2000), 65 (18): 5644-5646; and M. Okada et al., Planta Med. (2000), 66(6): 572-575.

When present, the coumestrol comprises greater than or equal to 0.5 weight percent, more preferably greater than or equal to about 1 weight percent, still more preferably greater than or equal to about 2 weight percent, even more preferably greater than or equal to about 5 weight percent, even more preferably greater than or equal to about 10 weight percent, still more preferably greater than or equal to about 20 weight percent of the total weight of the composition. Compositions containing as much as 50 weight percent, or even as much as 100 weight percent of coumestrol are contemplated.

Supplementary active ingredients may also be incorporated into the compositions and preparations. For example, administration of wogonin, isoliquiritigenin, coumestrol, and mixture comprising one or more of the foregoing phytoestrogens in combination with other anti-cancer agents is expected to stimulate anti-cancer activity.

In one embodiment, the composition may comprise: greater than or equal to 0.5 weight percent of a phytoestrogen selected from wogonin, isoliquiritigenin, coumestrol, and combinations comprising one or more of the foregoing phytoestrogens; and at least one anti-cancer agent In such compositions, the phytoestrogen selected from wogonin, isoliquiritigenin, coumestrol, and combinations thereof, may be present in an amount of about 0.5 to about 50 weight percent of the total weight of active ingredients in the composition. Within this range, the amount may be greater than or equal to about 1,2, 5, or 10 weight percent. Also within this range, the amount may be up to about 40, 30, or 20 weight percent. While the above weight percents are based on the total weight of active ingredients in the composition, they may, alternatively, be based on the total weight of phytoestrogen in the composition.

There is no particular limitation on the anti-cancer agent employed. Suitable anti-cancer agents include, for example, oridonin, indirubin, taxol, cis-platin, camptothecan, vincristine, monocrotaline, Maytansine, homoharringtonine, colchicine, irisquinone A, irisquinone B, irisquinone C, acronycine, matrin, oxymatrin, curcumin, paricine, pariphyllin, and the like, and combinations comprising one or more of the foregoing anti-cancer agents. Preferred anti-cancer agents include oridonin.

Suitable anti-cancer agents also include, for example, an extract of a plant selected from *Rabdosia rubescens, Panax pseudo-ginseng Wall, Ganoderma lucidum Karst, Scuttellaria baicalensis Georgi, Glycine max, Curcuma longa,* and the like, and combinations comprising one or more of the foregoing plants. An extract of *Rabdosia rubescens* may comprise oridonin; an extract of *Humulus lupulus* may comprise lupulone; an extract of *Panax pseudo-ginseng Wall* may comprise a gensenoside; an extract of *Scutellaria balcalensis Georgi* may comprise baicalin; an extract of *Glycine*

*max* may comprise a soy flavonoid, a soy isoflavonoid, or both; and an extract of *Curcuma longa* may comprise curcumin.

The anti-cancer agent may comprise, for example, about 1 to about 10 parts by weight of an extract of *Rabdosia rubescens;* about 10 to about 40 parts by weight of an extract of *Panax pseudo-ginseng Wall;* about 100 to about 500 parts by weight of an extract of *Ganoderma lucidum Karst;* about 10 to about 100 parts by weight of an extract of *Scutellaria baicalensis Georgi;* about 10 to about 100 parts by weight of an extract of *Glycine max;* and about 10 to about 100 parts by weight of an extract of *Curcuma longa.*

The anti-cancer agent may comprise, for example, an extract of *Humulus lupulus;* and an extract of a plant selected from the group consisting of *Panax pseudo-ginseng Wall, Ganoderma lucidum Karst, Scutellaria baicalensis Georgi, Glycine max, Curcuma longa,* and combinations comprising one or more of the foregoing plants.

The anti-cancer agent may comprise about 1 to about 10 parts by weight of an extract of *Humulus lupulus;* about 10 to about 40 parts by weight of an extract of *Panax pseudo-ginseng Wall;* about 100 to about 500 parts by weight of an extract of *Ganoderma lucidum Karst;* about 10 to about 100 parts by weight of an extract of *Scutellaria baicalensis Georgi;* about 10 to about 100 parts by weight of an extract of *Glycine max;* and about 10 to about 100 parts by weight of an extract of *Curcuma longa.*

The anti-cancer agent may be present at about 1 to about 90 weight percent of the total weight of active ingredients in the composition. Within this range, the anti-cancer agent amount may be greater than or equal to about 2, 5, or 10 weight percent. Also within this range, the anti-cancer agent amount may be up to about 80, 70, 50, or 25 weight percent.

In addition to an anti-cancer agent, the composition may, optionally, further comprise an immune stimulant. There is no particular limitation on the immune stimulant employed. Suitable immune stimulants include, for example, ginsenosides, ferulic acid, mannan, synanthrin, eleutheroside A, eleutheroside B, eleutheroside C, eleutheroside D, eleutheroside E, gynoside, beta-pachyman, inulin, glycoproteins, interferones, gamma-globulins, polysaccharides from *Ganoderma lucidum,* and the like, and combinations comprising one or more of the foregoing immune stimulants. Suitable immune stimulants further include, for example, extracts of *Ganoderma lucidum, Coriolus versicolor, Poria cocos,* and the like, and combinations comprising one or more of the foregoing extracts. Preferred immune stimulants include beta-pachyman.

The immune stimulant, when present, is employed at about 1 to about 90 weight percent o the total weight of active ingredients in the composition. Within this range, the immune stimulant amount may be greater than or equal to about 2, 5, 10, 20, or 50 weight percent. Also within this range, the immune stimulant amount may be up to about 80, 70, or 60 weight percent.

In a preferred embodiment, the composition comprises: greater than about 0.5 weight percent of a phytoestrogen selected from wogonin, isoliquiritigenin, coumestrol, or a combination comprising one or more of the foregoing compounds; an anti-cancer agent selected from oridonin, colchicine, vincristine, camptothecan, maytansine, taxol, and combinations comprising one or more the foregoing anti-cancer agents; and an immune stimulant selected from ginsenosides, mannan, synanthrin, eleutheroside A, eleutheroside B, eleutheroside C, eleutheroside D, eleutheroside E, gynosides, beta-pachyman, interferon, and combinations comprising one or more of the foregoing immune stimulants. In this embodiment, the composition preferably comprises: about 1 to about 40 weight percent of a compound selected from wogonin, isoliquiritigenin, coumestrol, and combinations comprising one or more of the foregoing compounds; about 0.05 to about 5 weight percent of a compound selected from oridonin, camptothecan, vincristine, Indirubin, colchicine, ginsenosides, and combinations comprising one or more of the foregoing compounds; and about 10 to about 98 weight percent of a compound selected from beta-pachyman, mannan, synanthrin, gynosides, and combinations comprising one or more of the foregoing compounds; wherein all weight percents are based on the total weight of the composition.

In another preferred embodiment, the composition comprises a phtoestrogen selected from the group consisting of wogonin, isoliquiritigenin, coumestrol and combinations comprising one or more of the foregoing compounds; oridonin; and beta-pachyman. In this embodiment, the composition preferably comprises: about 1 to about 30 weight percent of wogonin, isoliquiritigenin, coumestrol, or a combination comprising at least one of the foregoing compounds; about 0.1 to about 5 weight percent of oridonin; and about 20 to about 90 weight percent of beta-pachyman; wherein all weight percents are based on the total weight of the composition.

As the composition may be defined as comprising multiple components, it will be understood that each component is chemically distinct, particularly in the instance that a single chemical compound may satisfy the definition of more than one component.

Wogonin, isoliquiritigenin, coumestrol, their pharmaceutically acceptable salts or esters, or their selectively substituted analogs may be isolated from natural sources or synthesized according to known methods, as described above. Purities of these compounds, as employed in the composition, may vary according to their method of isolation or synthesis, but purities of about 5 percent to greater than 99 percent may be suitable for use in the composition.

The phytoestrogens may be in the form of a pharmaceutically acceptable composition. Methods for the formulation of pharmaceutically acceptable compositions are generally known. The subject pharmaceutical formulations may

comprise one or more non-biologically active compounds, i.e., excipients, such as stabilizers (to promote long term storage), emulsifiers, binding agents, thickening. agents, salts, preservatives, and the like, depending on the route of administration.

For oral administration, the wogonin, isoliquiritigenin, coumestrol, their pharmaceutically acceptable salts or esters, their selectively substituted analogs, or the like, or combinations comprising one or more of the foregoing may be administered with an inert diluent or with an assimilable edible carrier, or incorporated directly with the food of the diet. The formulations may be incorporated with excipients and used in the form of ingestible tablets, buccal tablets, troches, capsules, elixirs, suspension syrups, wafers, and the like. The tablets, troches, pills, capsules and the like may also contain the following: a binder, such as gum tragacanth, acacia, cornstarch, or gelatin; excipients, such as dicalcium phosphate, a disintegrating agent such as corn starch, potato starch, alginic acid and the like; a lubricant such as magnesium stearate; and a sweetening agents, such as sucrose, lactose or saccharin; a flavoring agent such as peppermint, oil of wintergreen, or the like flavoring. When the dosage unit is a capsule, it may contain, in addition to materials of the above type, a liquid carrier. Various other materials may also be present as coatings or to otherwise modify the physical form of the dosage unit. A syrup or elixir may contain sucrose as a sweetening agent, methyl and propylparabens as preservatives, a dye and flavoring such as cherry or orange flavor. Such additional materials should be substantially non-toxic in the amounts employed. Furthermore, the active agents may be incorporated into stained-release preparations and formulations. Formulations for parenteral administration may include sterile aqueous solutions or dispersions, and sterile powders for the extemporaneous preparation of sterile, injectable solutions or dispersions. The solutions or dispersions may also contain buffers, diluents, and other suitable additives, and may be designed to promote the cellular uptake of the active agents in the composition, e.g., liposomes. Sterile injectable solutions are prepared by incorporating the active compounds in the required amount in the appropriate solvent with one or more of the various other ingredients described above, followed by sterilization. Dispersions may generally be prepared by incorporating the various sterilized active ingredients into a sterile vehicle that contains the basic dispersion medium and the required other ingredients from those listed above. In the case of sterile powders used to prepare sterile, injectable solutions, the preferred methods of preparation are vacuum-drying and freeze-drying techniques which yield a powder of the active ingredient plus any additional desired ingredient from previously sterile-filtered solutions. Pharmaceutical formulations for topical administration may be especially useful for localized treatment. Formulations for topical treatment included ointments, sprays, gels, suspensions, lotions, creams, and the like. Formulations for topical administration may include known carrier materials such as isopropanol, glycerol, paraffin, stearyl alcohol, polyethylene glycol, and the like. The pharmaceutically acceptable carrier may also include a known chemical absorption promoter. Absorption promoters include, for example, dimethylacetamide (U.S. Pat. No. 3,472,931 to Stoughton), trichloroethanol or trifluoroethanol (U.S. Pat. No. 3,891,757 to Higuchi), certain alcohols and mixtures thereof (British Patent Nos. 1,001,949 to Meyer and 1,464,975 to Astra Lakemedel). Except insofar as any conventional media or agent is incompatible with the therapeutic active ingredients, its use in the therapeutic compositions and preparations is contemplated.

The composition may, optionally, be in an ingestible form, preferably a powder, a capsule, or a tablet. Alternatively, the composition may be in the form of a suppository.

The pharmaceutical compositions described preferably contain about 0.5% to 100% by weight of active agent. Within this range, the compositions and preparation may preferably comprise the active agent in an amount of at least about 1, 2, 5, 10, or 20 weight percent. Also within this range, the composition may preferably comprise the active agent in an amount of up to about 90, 80, 70, 60, or 50 weight percent. The amount of active compounds in such pharmaceutically useful compositions and preparations is such that a suitable dosage will be obtained.

Another embodiment is a method for the treatment of a human having cancer, an estrogen-related cancer, or other estrogen-related disorder. The method comprises treating a human in need of such treatment with a composition comprising a phytoestrogen selected from wogonin, isoliquiritigenin, coumestrol, or a combination comprising one or more of the foregoing phytoestrogens. The terms "treating" and "treatment" as used herein refer to reduction in severity and/or frequency of symptoms, elimination of symptoms and/or underlying cause, prevention of the occurrence of symptoms and/or their underlying cause, and improvement or remediaton of damage. Thus, for example, the present method of "treating" an estrogen-dependent disorder or cancer, as the term is used herein, encompasses both prevention of the disorder and treatment of the disorder in a clinically symptomatic individual.

By the terms "effective amount" or "pharmaceutically effective amount" or "an effective anti-estrogenic amount" of an agent as provided herein are meant a nontoxic but sufficient amount of the agent to provide the desired prophylactic or therapeutic effect. As will be pointed out below, the exact amount required will vary from subject to subject, depending on the age and general condition of the subject, the severity of the condition being treated, and the particular phytoestrogen employed and mode of administration, and the like. Thus, it is not possible to specify an exact "effective amount". However, an appropriate "effective" amount in any individual case may be determined by one of ordinary skill in the art using only routine experimentation.

By "pharmaceutically acceptable carrier" is meant a material which is not biologically or otherwise undesirable, i.e., the material may be administered to an individual along with the selected phytoestrogen without causing any undesirable biological effects or interacting in a deleterious manner with any of the other components of the pharmaceutical composition in which it is contained.

Cancer is the growth of new cells in the body wherein the new cells typically have adverse effects in the body. Cancer is characterized by an increase in the number of abnormal, or neoplastic, cells derived from a normal tissue which proliferate to form a tumor mass, the invasion of adjacent tissues by these neoplastic tumor cells, and the generation of malignant cells which eventually spread via the blood or lymphatic system to regional lymph nodes and to distant sites via a process called metastasis. In a cancerous state, a cell proliferates under conditions in which normal cells would not grow. Cancer manifests itself in a wide variety of forms, characterized by different degrees of invasiveness and aggressiveness.

Administration of a phytoestrogen such as wogonin, isoliquiritigenin, coumestrol, or combinations thereof is effective to provide anti-cancer activity. It is believed that the general anti-cancer activity of wogonin, isoliquiritigenin, coumestrol is related to their activity as COX-2 inhibitors. COX-2 is a key inducible enzyme in the conversion of arachidonic acid to prostaglandins and other eicosanoids. COX-2 expression can be induced by a variety of factors; including, for example, growth factors, interleukin-1, and tumor promoting factors. The enzyme is expressed in a number of tumor cells, and human cancers, among which is prostate cancer. COX-2 inhibitors are known have use as anti-cancer therapeutics.

In addition to general anti-cancer activity, the phytoestrogens wagonin, isoliquiritigenin, and coumestrol are useful as anti-hormone-related cancer agents. Hormone-rolated cancers include, for example, bladder cancer, bone cancer, breast cancer colon cancer, endometrial cancer, lung cancer, ovarian cancer, prostate cancer, testicular cancer, and thyroid cancer.

In the treatment of cancer, the phytoestrogen compositions may be administered orally, parenterally, transdermally, rectally, nasally, buccally, vaginally or via an implanted reservoir in dosage formulations containing conventional non-toxic pharmaceutically acceptable carriers, adjuvants and vehicles. The term "parenteral" as used herein is intended to include subcutaneous, intravenous, and intramuscular injection. The amount of active compound administered will, of course, be dependent on the subject being treated, the subject's weight, the manner of administration and the judgment of the prescribing physician. Generally, however, the dosage of phytoestrogen will be about 0.01 mg/kg/day to about 1000 mg/kg/day, preferably about 0.01 mg/kg/day to about 300 mg/kg/day, more preferably about 1 mg/kg/day to about 300 mg/kg/day. When the phytoestrogen is used in combination with an anti-cancer agent, the dosage of the phytoestrogen will be about 0.01 mg/kg/day to about 1000 mg/kg/day, preferably about 0.01 mg/kg/day to about 300 mg/kg/day, more preferably about 1 mg/kg/day to about 300 mg/kg/day, and the dosage of anti-cancer agent will be about 0.1 ug/kg/day to about 100 mg/kg/day, preferably about 0.3 ug/kg/day to about 50 mg/kg/day, more preferably about 0.01 mg/kg/day to about 50 mg/kg/day. When the phytoestrogen composition comprises an immune stimulant, the dosage of immune stimulant will be about 1 mg/kg/day to about 5000 mg/kg/day, more preferably about 5 mg/kg/day to about 1000 mg/kg/day

The phytoestrogens wogonin, isoliquiritigenin, and coumestrol may also be used in the treatment of other estrogen-related disorders including, for example, bone loss, bone fractures, osteoporosis, glucocorticoid induced osteoporosis, Paget's disease, abnormally increased bone turnover, periodontal disease, tooth loss, rheumatoid arthritis, osteoarthritis, periprosthetic osteolysis, osteogenesis imperfecta, metastatic bone disease, hypercalcemia of malignancy, cartilage degeneration, endometriosis, uterine fibroid disease, hot flashes, cardiovascular disease, impairment of cognitive function, cerebral degenerative disorders, restenosis, gynecomastia, vascular smooth muscle cell proliferation, obesity, incontinence, and combinations thereof. As used herein, estrogen-related disorder also includes the symptoms of menopause, the transition from the reproductive stage to the non-reproductive stage of a woman's life, characterized primarily by the cessation of menstruation. Symptoms of menopause include, for example, hot flashes, sweating secondary to vasomotor instability, psychological and emotional symptoms of fatigue, insomnia, irritability and nervousness, lack of sleep, dizziness, cardiac symptoms; the incidence of heart disease increases, nausea, constipation, diarrhea, arthralgia, myalgia, and combinations of the foregoing symptoms.

Of particular interest is the treatment and prevention of osteoporosis. Osteoporosis, or loss of bone density, results in increased bone fractures and vertebral column collapse. Bone loss often begins around age 35. This loss accelerates during menopause, which generally occurs around age 45 to 55. Bone mass losses average about 1-2% each year after menopause. The primary sites are the vertebrae, which show anterior collapse resulting in stooping and backache, the hips and the wrist. Osteoporosis develops over decades and is related to peak bone mass, as well as to the degree of bone loss.

In the treatment of an estrogen-related disorder, the phytoestrogen compositions may be administered orally, parenterally, transdermally, rectally, nasally, buccally, vaginally or via an implanted reservoir in dosage formulations containing conventional non-toxic pharmaceutically acceptable carriers, adjuvants and vehicles. The term "parenteral" as used herein is intended to include subcutaneous, intravenous, and intramuscular injection. The amount of active compound administered will, of course, be dependent on the subject being treated, the subject's weight, the manner of administration and the judgment of the prescribing physician. Generally, however, the dosage of phytoestrogen will be

about 0.1 mg/kg/day about 1000 mg/kg/day, more preferably about 0.1 mg/kg/day to about 300 mg/kg/day.

The invention is further ilustrated by the following non-limiting examples.

GENERAL EXPERIMENTAL

Wogonin was extracted from 7 grams of a multi-component botanical extract composition containing extracts of Panax pseudo-ginseng Wall, Isatis Indigotica Fort, Ganoderma lucidium Karst, Dendrathema morifolium Tzvel, Glycyrrhiza glabra L, Sculletaria bailcalensis Georgi, Rabdosia rubescens, and Screnoa repens. The powder was dissolved in 150 milliliters of acetone using a soxhlet extractor for one hour. The liquid phase extract was further purified by silica gel column chromatography using a solvent system of 2:1 cyclohexane:acetone. About 25 milligrams of the yellow powder was obtained from fractions 11-13 (8 milliliters per fraction). The powder was further re-crystallized from absolute ethanol to yield yellow crystals.

Figure 1 is a high performance liquid chromatogram showing the location of wogonin in the elution profile. The chromatogram was obtained with a Shimadzu SPD-M10A chromatograph using a C18 reverse phase column and two solvent systems of water and acetonitrile in 0.1% trifluoracetic acid.

The chemical structure and molecular weight of the yellow crystal was determined by total (Fig. 2(b)) and DEPT (Fig. 2(a)) $^{13}$C NMR (d-4 methanol solvent analyzed on Varian$^{UNITY}$ Inova 400 system). The sample was also analyzed by electrozlionization with a Hewlet Packard VG 7070; the mass spectrum is shown in Figure 3 and indicates a molecular weight of 284, consistent with wogonin.

Isoliquiritigenin was purified from the flavonoid fraction of *Glycyrrhiza glabra* concentrated powder (purchased from Shanghai Zhao Wei Technology Development Co.) extracted by absolute ethanol: About 1 gram of the flavonoid concentrate (dissolved in 5 milliliters of water) was passed through Sephadex LH-20 column (2.5x30 millimeters) and eluted by a gradient of methanol-water mixed solvent. Crude isoliquiritigenin was obtained at fraction 27 (10 milliliters per fraction). The crude product was further purified by silica gel chromatography eluted by mixed solvent of methylene chloride: methanol (5:1).

The chemical structure and the molecular weight of isoliquiritigenin were determined by the absorption spectrum (Fig. 5) associated with an HPLC separation (Fig. 4) performed on a Sbimadzu SPD-M10A chromatograph, as well as the $^{13}$C NMR spectra (Fig. 6(a), DEPT; Fig. 6(b), total) and mass spectrum (Fig. 7). The absorption spectrum was identical to that of the reference compound isoliquiritigenin purchased from Sigma Chemical Co, (St. Louis, Missouri).

The anti-cancer activities of wogonin and isoliquiritigenin were evaluated by determining their abilities to inhibit cancer cell growth, to modulate the cancer cell cycle, and to activate estrogen receptors.

Cancer cell lines: LNCaP, DU-145, and MCF-7 cells were purchased from the American Type Culture Collection, PTX10, a taxol-resistant ovarian cancer cell line, was obtained from the Brander Cancer Research Laboratory, New York Medical College. Cells were maintained in RPMI 1640 culture media supplemented with 10% heat-inactivated FBS, 5 millimolar glutamine, 50 units/milliliter of penicillin G, and 50 grams/milliliter of streptomycin. The cells were routinely seeded at 1X 105 cells/milliliter in T-75 flasks, allowed to attach overnight, then treated with the herbal extract. At different times, cells were harvested by trypsinization.

EXAMPLE 1

This example demonstrates the activity of wogonin and isoliquiringenin in inhibiting the growth of the hormone-sensitive prostate cancer cell line, LNCaP.

The MTT assay (MTT = 3-(4,5-dimethylthiazol-2-yl)-2,5-diphenyl tetrazolium bromide) was used to count viable cells. The assay reagents were purchased from Boehringer Mannheim (Roche Diagnosis Corp, Indianapolis, Indiana). In this assay, the tetrazolium dye MTT is cleaved to form formazan by metabolically active cells and exhibits a strong red absorption band at 550-618 nm. The protocol for the cell viability assay was provided by the manufacturer and modified in our laboratory as described below.

Prostate or breast cancer cells were seeded in 96 well microtiter plates at a concentration of $3x10^3$ cells per well (MCF-7; breast cancer cells) or $6x10^3$ cells per well (LNCaP or DU145; prostate cancer cells), in a volume of 100 microliters of cell culture medium. After 24 hours, 20 microliter aliquots of the compounds at various concentrations were added to the attached cells. Each concentration was repeated in 3, different wells to obtain mean values. To eliminate any solvent effect, 20 microliters of the solvent used in the preparation of the highest concentration of the compounds (a maximum of 0.5 % by volume of dimethylsulfoxide (DMSO)) was added to the control cells in each well. The plates were incubated at 37°C in a $CO_2$ incubator for 72 hours. At the end of day 3 (after 72 hours), the culture medium was carefully removed without disturbing the cells and replaced by 100 microliters of fresh cell , medium. Ten microliters of MTT reagent was added to each well and the plates were incubated again in a $CO_2$ incubator at 37°C for 4 hours. One hundred microliters of sodium dodecylsulfate (SDS) solubilizing reagent (from Boehringer Mannheim) was added to each well. The plate was allowed to stand overnight in the $CO_2$ incubator , and read by an ELISA Reader (EL800) Bio-Tek Instruments, Inc.) at a wavelength of 570 nm. The percent cell viability was calculated according to the equation below:

$$V = 100\left(\frac{A_{control} - A_{treated}}{A_{control}}\right)$$

where $V$ is the percent cell viability, $A_{control}$ is the absorption of the control cells, and $A_{treated}$ is the absorption of the treated cells. Figures 8 and 9 demonstrate the activity of wogonin and isoliquiritigenin respectively, in inhibiting the growth of LNCaP (androgen-dependent) and DU-145 (androgen-independent) prostate cancer cells. It is apparent that the inhibition of cell growth is dose dependent. The concentration of the compounds resulting in 50% inhibition of cancer cell growth, defined as $ED_{50}$, was determined by linear interpolation. $ED_{50}$ values for the two compounds obtained from these measurements are shown in Columns 1 and 2 of Table 1.

Table 1. $ED_{50}$ values from MTT Assay as a Function of Compound and Cell Type

| | LNCaP (μg/ml) | DU145 (μg/ml) | MCF7 (μg/ml) |
|---|---|---|---|
| Wogonin | 10.00 | 18.6 | NA |
| isoliquiritigenin | 3.51 | 7.60 | 3.25 |

EXAMPLE 2

This example demonstrates the activity of isoliquiritigenin in inhibiting the growth of the breast cancer cell line, MCF-7.

The same protocols described in Example 1 were used to evaluate the effects of isoliquiritigenin on MCF-7 cells. MCF-7 is a breast cancer cell line that expresses estrogen receptors. Therefore it is a good model to study the effect of the anti-cancer agents on estrogen-receptor positive breast cancer.

Figure 9 shows the MTT assay curves for isoliquiritigenin with MCF-7. The data show that isoliquiritigenin inhibited the growth of MCF-7 cells, and dosage-dependent curves were observed. $ED_{50}$ values are given in Column 3 of Table 1, above.

EXAMPLE 3

This example demonstrates modulation of the LNCaP cell cycle by wogonin and isoliquiritigenin. LNCaP cells have a hormone-dependent cell cycle.

Sample preparation for cell cycle measurement: Cultured cells (2-4 x $10^6$ cells) were exposed to two concentrations each of wogonin and isoliquiritigenin for 24-48 hours in 12.5 $cm^2$ area flasks before being harvested. The cells were washed with phosphate buffered saline (PBS) and fixed in ice-cold 70% ethanol. Aliquots of fixed cells were rehydrated in PBS and stained with 1.0 microgram/milliliter DAPI (4,6-diamidino-2-phenylindole from Eastman Kodak, Rochester, NY), and dissolved in 10 millimolar piperazine-NN-bis-2-ethane-sulfonic acid buffer (Calbiochem, La Jolla, CA) containing 100 millimolar NaCl, 2mM $MgCl_2$ and 0,1% Triton X-100 (Sigma) at pH 6.8 as previously described by Halicka et al. (H. D. Halicka, B. Ardelt, G. Juan, A. Mittelman, S. Chen, F. Traganos and Z. Darzynkiewicz, "Apoptosis and Cell Cycle Effects Induced by Extracts of the Chinese Herbal Preparation of PC SPES", International J. of Oncology (1997), 11: 437-448).

The cellular DNA content was measured with an ELITE ESP flow cytometer (Coulter Inc., Fl.) using UV laser illumination. The multicycle program was used to deconvolute the DNA frequency histograms to estimate the frequency of cells in different phases of the cell cycle.

Figure 10 displays the DNA histograms of LNCaP cells in the presence and absence of wogonin at 3 micrograms/milliliter after 24 hours. It is evident that there was a change at the G1 phase as shown by the arrow bar. Data analysis revealed the increase in G1 phase was proportional to wogonin concentration.

Similar measurements were conducted for isoliquiritigenin. Figure 11 summarizes the effects of wogonin and isoliquiritigenin on G1, S, and $G_2M$ phases of the LNCaP cell cycle. The data show that wogonin induced a G1 phase arrest, and isoliquiritigenin induced a $G_2M$ phase arrest. A prolongation in either G1 or $G_2M$ phases leads to the suppression of LNCaP cell proliferation.

EXAMPLE 4

This example demonstrates modulation of the DU-145 cell cycle by wogonin and isoliquixitigenin.

The protocol described in Example 3 was used to study the effect of wogonin on the hormone-independent prostate cancer cell line DU-145. The results are presented in Figure 12 and show that wogonin prolonged the $G_2M$ phase of DU-145. A prolongation in the $G_2M$ phase leads to the suppression of DU-145 cell proliferation.

EXAMPLE 5

The estrogenic activity of wogonin and isoliquiritigenin were demonstrated by determining their ability to activate estrogen receptors (subclass alpha and beta).

HEK 293 cells (ATCC CRL-1573) were transfected with an expression vector for $hER_{\alpha}$ and $hER_{\beta}$ respectively, and an ERE-LUC reporter gene (plus a TK-LUC reporter for normalization) following the protocol of Yoon et al ("Differential activation of wild-type and variant forms of estrogen receptor $\alpha$ by synthetic and natural estrogenic compounds using a promoter containing three estrogen-responsive elements", J. Steroid Biochem. & Molecular Biology (2000), 28: pages 25-32).

Cells were then separately exposed to wogonin or isoliquiritigenin at concentrations of 0, 0.07, 0.02, 0.08, 0.3, 0.7, 1.4 (or 4) and 9 μg/ml for 20 hours and then cell lysates were assayed for reporter gene expression (Tzukerman et al. "Human estrogen receptor transactivational capacity is determined by both cellular and promoter content and mediated by two functionally distinct intermolecular regions", Mol. Endocrinol. (1994), 8: 21-30).

Figure 13 shows the dose-responsive behavior of ERα-Luc reporter gene activated by wogonin and isoliquiritigenin.

Figure 14 shows the dose-responsive behavior of ERβ-Luc reporter gene activated by wogonin and isoliquiritigenin. It is very significant that wogonin and isoliquiritigenin showed at least 10 times more capability in activating ERβ-Luc reporter gene than the ERα-Luc reporter gene.

EXAMPLE 6

This example demonstrates the inhibition of COX-2 activity by isoliquiritigenin.

COX is a bifunctional enzyme that exhibits both cyclooxygenase and peroxidase activities. The cyclooxygenase activity is responsible for the oxidation of arachidonic acid to Prostaglandin $G_2$ ($PGG_2$) and the peroxidase activity is responsible for the subsequent reduction of $PGG_2$ to the corresponding alcohol, $PGH_2$. Some methods used to determine COX inhibitor activity include measuring uptake of oxygen using an oxygraph, measuring the conversion of radioactive arachidonic acid, or measuring the prostaglandins formed from $PGH_2$ (using immunoassay techniques).

These experiments employed the ovine COX-2 Inhibitor Screening Assay commercially available as catalog no. 760101 from Cayman Chemical (Ann Arbor, MI 48108). This immunoassay uses an antibody to that binds all major prostaglandins to measure quantitatively the amount of $PCF_{2\alpha}$ produced in the COX reaction using arachidonic acid as a substrate. The final volume of the reactions as described below is typically 1.15 ml. In brief, COX-1 or COX-2 (e.g., 20 μl of a solution of from 1 to 100 units/ml COX, where a unit is defined as the amount of enzyme that consumes one nanomole of oxygen per minute at 37°C in 0.1 M Tris-HCl buffer, pH 8.0 containing 100 μM arachidonate, 5 mM EDTA, 2 mM phenol and 1 μM hematin) is first mixed with a buffer (e.g., 0.1 M Tris, pH 8.0, 5 mM EDTA, 2 mM phenol) and heme (e.g., 10 μl of 10 mM heme) in a microfuge tube. The heme is a cofactor for COX that provides maximal activity in the assay, COX samples that contain an inhibitor can be pre-mixed with the inhibitor (e.g., 1-100 μM) prior to adding substrate. The arachidonic acid substrate (e.g., 10 μl of a 10 mM solution) can be then added to the COX/heme/inhibitor mixture for a time and at a temperature sufficient for the reaction to proceed to produce a detectable product (e.g., 2 minutes at 37°C). The reaction can be quenched with acid (e.g., 50 μl of 1 M. HCl). Additionally stannous chloride (e.g., 100 μl of a saturated solution) can be added to convert the $PGH_2$ produced to the more stable $PGF_{2\alpha}$ for the purpose of quantification of prostaglandin. The prostaglandin produced in the reactions is typically quantified using an enzyme immunoassay.

The enzyme immunoassay to detect prostaglandin can conveniently be performed in a 96 well plate using an antibody to detect prostaglandin. As controls, prostaglandin standards and COX 100% activity samples (no inhibitor) can be measured. Samples used for background correction can also be used. Controls and reactions performed with COX and the inhibitors of interest are incubated with prostaglandin screening antiserum in an amount sufficient to detect the prostaglandin produced in the COX reactions (e.g., 50 μl of antiserum diluted in 6 ml of a suitable EIA buffer). The reactions can be incubated for a time and temperature sufficient to allow interaction of the antiserum and the prostaglandins (e.g., 18 hours at room temperature). When ready to develop the plate, the reactions are first washed and then incubated with 200 μl of Ellman's reagent for 60 minutes or so. The plate can be read at 405 to 420 nm on a plate reader. The prostaglandin standards are used to calculate a standard curve of prostaglandin concentration. The amount of

prostaglandin in each sample with inhibitor is subtracted from the amount of prostaglandin in the 100% activity sample, divided by the amount of prostaglandin in the 100% activity sample, and multiplied by 100 to give the percent inhibition. Graphing the percent inhibition vs. the inhibitor calculation allows the calculation of the $IC_{50}$ value (the concentration at which there is 50% inhibition).

Methods of measuring the activity of a COX inhibitor are described in, for example, W. Xie, J. G. Chipman, D. L, Robertson, et al., "Expression of a mitogen-responsive gene encoding prostaglandin synthase is regulated by mRNA splicing", Proc. Natl. Acad. Sci.USA (1991), 88: 2692-2696; K.M. Maxey, K.R. Maddipati, and J. Birkmeier, "Interference in enzyme immunoassays", J. Clin. Immunoassay (1992), 15: 116-120; P. Pradelles, J. Grassi, and J. A. Maclouf, "Enzyme immunoassays of eicosanoids using acetylcholinesterase as label: An alternative to radioimmunoassay", Anal. Chem. (1985), 57: 1170-1173; Maclouf J., Grassi, J., and Pradelles, P. Development of enzyme-immunoassay techniques for the measurement of eicosanoids, Prostaglandin and Lipid Metabolism in Radiation Injury (1987), pages 355-364.

Figure 15 displays the dose-dependent inhibitory activity of isoliquiritigenin on COX-2 measured according to the procedure above, An $IC_{50}$ of 10.5 $\mu$M was calculated from the data.

EXAMPLE 7: demonstrates the cytotoxicity of wogonin and isoliquiritigenin on an ovarian cancer cell line:

PTX 10, a taxol resistant ovarian cancer cell line, cells were maintained in RPMI 1640 medium supplemented with 10% fetal calf serum, 100 units/ml penicillin, 100 mg/ml streptomycin, and 2mM L-glutamine (all from Gibco/BRL Life Technologies, Inc., Grand Island, NY) at 37,5°C in an atmosphere of 5% $CO_2$ in air. At the onset of experiments the cultures were at the densities below $5x10^5$ cells/ml and the cells were growing exponentially and asynchronously.

The MTT assay was performed to study the effect of wogonin and isoliquiritigenin on the cell growth of PTX 10. The MTT assay protocol is the same as that described in Example 1. Figures 16 and 17 display the inhibition curves of PTX cell line in the presence of wogonin and isoliquiritigenin respectively. A concentration dependent inhibition was clearly observed in these two figures. The $ED_{50}$ calculated from the inhibition curves are 1.56 ug/mL and 3.32 ug/mL for wogonin and isoliquiritigenin respectively as shown in Table 2.

Table 2 $ED_{50}$

| | PTX 10 (taxol resistant Ovarian cancer cell) |
|---|---|
| Wogonin (I-16-2) | <1.56ug/mL |
| Isoliquiritigenin | 3.32ug/ml |

Thus, wogonin and isoliquiritigenin may have utility for treating cancers that are resistant to treatment by other agents such as, for example, taxol.

EXAMPLE 8

A composition according to this disclosure was administered in capsules 6 times a day to two elderly volunteer patients diagnosed with prostate cancer. As a measure of the progress of the cancer, the bloodstream level of prostate-specific antigen (PSA), a substance produced by the prostate gland, was measured by standard methods. The results are shown in Table 3.

Table 3

| Patient Age | Treatment prior to treatment with Composition 1 | PSA at start of treatment, ng/mL | PSA after 1 month of treatment, ng/mL | PSA after 2 months of treatment, ng/mL | Percent PSA reduction after 1 month of treatment | Percent PSA reduction after 2 months of treatment |
|---|---|---|---|---|---|---|
| 73 | Castration plus Flutamide | 80 | 40 | 6 | 50% | 92.5% |
| 79 | Prostatectomy; no medication | 120 | 64 | 18 | 46.7% | 85% |

As can be seen from table 1; a dramatic reduction in PSA levels is observed after 1 and 2 months of treatment with composition 1.

Compositions comprising wogonin, isoliquiritigenin, coumestrol and combinations thereof have both phytoestrogenic and anti-cancer activities. When used in cancer therapy, the compositions may comprise additional anti-cancer agents and/or immune stimulants. The anti-cancer activity is demonstrated by the ability of wogonin and isoliquiritigenin to inhibit the growth of cancer cell lines. The identification of isoliquiritigenin as a COX-2 inhibitor suggests that it has general anti-cancer activity. The identification of the phytoestrogenic activity of wogonin suggests that, in addition to general anti-cancer activity due to COX-2 inhibition, wogonin may be particularly useful in the treatment of hormone-related cancers. In addition, wogonin may be used in the treatment of hormone-related disorders such as, for example, osteoporosis.

While the invention has been described with reference to a preferred embodiment, it will be understood by those skilled in the art that various changes may be made and equivalents may be substituted for elements thereof without departing from the scope of the invention. In addition, many modifications may be made to adapt a particular situation or material to the teachings of the invention without departing from essential scope thereof. Therefore, it is intended that the invention not be limited to the particular embodiment disclosed as the best mode contemplated for carrying out this invention, but that the invention will include all embodiments falling within the scope of the appended claims.

All cited patents, patent applications, and other references are incorporated herein by reference in their entirety.

## Claims

1. A method of treating a human in need of cancer treatment, comprising administering a composition comprising greater than 0.5 weight percent based on the total weight of the composition of wogonin, a pharmaceutically acceptable salt or ester of wogonin, a selectively substituted analog of wogonin, or a combination of one or more of the foregoing compounds.

2. The method of Claim 1, wherein the composition comprises a glycoside of wogonin; a selectively substituted analog of wogonin selected from the group consisting of genistein, biochanin, prunetin, scutellarein, daidzin, luteolin, apigenin, acacetin, 3,6, 4-trihydoxylflavone, 7,3-dihydroxy-4,1-dimethoxy-isoflavone, 3R-2', 3'-dihydoxy-7, 4-dimethoxy-isoflavone; or a combination of one or more of the foregoing compounds.

3. The method of Claim 1, wherein the composition comprises an extract of an herb in the family *Scutellaria.*

4. The method of Claim 1, wherein treating comprises administering a dosage of about 0.001 mg/kg/day to about 300 mg/kg/day of wogonin.

5. The method of Claim 1, wherein the composition further comprises isoliquiritigenin, coumestrol, or a combination of one or more of the foregoing compounds.

6. A method of treating a human in need of cancer treatment, comprising administering a composition comprising greater than 0.5 weight percent based on the total weight of the composition of wogonin, isoliquiritigenin, coumestrol, their pharmaceutically acceptable salts and esters, their selectively substituted analogs, or " a combination of one or more of the foregoing compounds.

7. The method of Claim 1, wherein the cancer is prostate cancer, breast cancer, endometrial cancer, colon cancer, lung cancer, bladder cancer, testicular cancer, ovarian cancer, thyroid cancer, or bone cancer.

8. The method of Claim 6, wherein the composition comprises a compound selected from the group consisting of isoliquiritigenin, a pharmaceutically acceptable salt or ester of isoliquiritigenin, a selectively substituted analog of isoliquiritigenin, or a combination of one or more of the foregoing compounds.

9. The method of Claim 8, wherein the composition comprises phloretin, 4,2, 4'-trihydroxychalcone, or a combination of one or more of the forgoing compounds.

10. The method of Claim 8, wherein the composition comprises an extract of *Glycyrrhiza uralensis, Glycyrrhiza glabra* or a combination of one or more of the foregoing compounds.

11. The method of Claim 8, wherein the composition further comprises wogonin, coumestrol, or a combination of one or more of the foregoing compounds.

**12.** The method of Claim 8, wherein treating comprises administering a dosage of about 0.001 mg/kg/day to about 300 mg/kg/day of isoliquiritigenin.

**13.** The method of Claim 6, wherein the composition comprises a compound selected from the group consisting of coumestrol, a pharmaceutically acceptable salt or ester of coumestrol, a selectively substituted analog of coumestrol, a, or a combination of one or more of the foregoing compounds.

**14.** The method of Claim 13, wherein the composition comprises an extract of a plant selected from the group consisting of *Taraxacum mongolicum, Medicago sativa, Brassica oleracea,* or *Eclipta prostrata* and combinations of one or more of the foregoing plant extracts.

**15.** The method of Claim 13, wherein the composition further comprises wogonin, isoliquiritigenin, or a combination of one or more of the foregoing compounds.

**16.** The method of Claim 13, wherein treating comprises administering a dosage of about 0. 001 mg/kg/day to about 300 mg/kg/day of coumestrol.

**17.** The method of Claim 1 or 6, wherein the composition further comprises an anti-cancer agent.

**18.** The method of Claim 17, wherein the anti-cancer agent is selected from the group consisting of oridonin, indirubin, taxol, cis-platin, camptothecan, vincristine, monocrotaline, Maytansine, homoharringtonine, colchicine, irisquinone A, irisquinone B, irisquinone C, acronycine, matrin, oxymatrin, curcumin, paricine, pariphyllin, and combinations comprising one or more of the foregoing anti-cancer agents.

**19.** The method of Claim 17, wherein the composition further comprises an immune stimulant

**20.** The composition of Claim 19, wherein the immune stimulant is selected from the group consisting of ginsenosides, ferulic acid, mannan, synanthrin, eleutheroside A, eleutheroside B, eleutheroside C, eleutheroside D, eleutheroside E, gynoside, beta-pachyman, inulin, glycoproteins, interferones, $\gamma$-globulins, extracts of *Ganoderma lucidum,* extracts of *Coriolus versicolor,* extracts of *Poria cocos,* and combinations comprising one or more of the foregoing immune stimulants.

**21.** A composition, comprising:

greater than or equal to about 0.5 weight percent based on the total weight of the composition of a compound selected from wogonin, its pharmaceutically acceptable salts or esters, its selectively substituted analogs, and combinations comprising one or more of the foregoing compounds; and
at least one anti-cancer agent.

**22.** A composition, comprising:

greater than or equal to about 0.5 weight percent based on the total weight of the composition of a compound selected from wogonin, isoliquiritigenin, coumestrol; their pharmaceutically acceptable salts or esters, their selectively substituted analogs, and combinations comprising one or more of the foregoing compounds; and
at least one anti-cancer agent.

**23.** The composition of Claim 22, wherein the anti-cancer agent is selected from the group consisting of oridonin, indirubin, taxol, cis-platin, camptothecan, vincristine, monocrotaline, Maytansine, homoharringtonine, colchicine, irisquinone A, irisquinone B, irisquinone C, acronycine, matrin, oxymatrin, curcumin, paricine, pariphyllin, and combinations comprising one or more of the foregoing anti-cancer agents.

**24.** The composition of Claim 22, wherein the anti-cancer agent comprises: an extract of Rabdosia rubescens; and an extract of a plant selected from the group consisting of Panax pseudo-ginseng Wall, Ganoderma lucidum Karst, Scutellaria baicalensis Georgi, Glycine max, Curcuma longa, and combinations comprising at least one of the foregoing plants.

**25.** The composition of Claim 24, wherein the anti-cancer agent comprises:

about 1 to about 10 parts by weight of an extract of *Rabdosia rubescens;*
about 10 to about 40 parts by weight of an extract of *Panax pseudo-ginseng Wall;*
about 100 to about 500 parts by weight of an extract of *Ganoderma lucidum Karst;*
about 10 to about 100 parts by weight of an extract of *Scutellaria baicalensis Georgi;*
about 10 to about 100 parts by weight of an extract of *Glycine* max; and
about 10 to about 100 parts by weight of an extract of *Curcuma donga.*

**26.** The composition of Claim 22, wherein the anti-cancer agent comprises:

an extract of *Humulus lupulus;* and an extract of a plant selected from the group consisting of *Panax pseudo-ginseng Wall, Ganoderma lucidum Karst, Scutellaria baicalensis Georgi, Glycine max, Curcuma longa,* and combinations comprising one or more of the foregoing plants.

**27.** The composition of Claim 26, wherein the anti-cancer agent comprises:

about 1 to about 10 parts by weight of an extract *of Humulus lupulus;*
about 10 to about 40 parts by weight of an extract of *Panax pseudo-ginseng Wall;*
about 100 to about 500 parts by weight of an extract of *Ganoderma lucidum Karst;*
about 10 to about 100 parts by weight of an extract of *Scutellaria baicalensis Georgi;*
about 10 to about 100 parts by weight of an extract of *Glycine max;* and
about 10 to about 100 parts by weight of an extract of *Curcuma longa.*

**28.** The composition of Claim 22, further comprising an immune stimulant.

**29.** The composition of Claim 28, wherein the immune stimulant is selected from the group consisting of ginsenosides, ferulic acid, mannan, synanthrin, eleutheroside A, eleutheroside B, eleutheroside C, eleutheroside D, eleutheroside E, gynoside, beta-pachyman, inulin, glycoproteins, interferones, $\gamma$-globulins, extracts of *Ganoderma lucidum,* extracts of *Coriolus versicolor,* extracts *of Poria cocos,* and combinations comprising one or more of the foregoing immune stimulants.

**30.** A composition, comprising:

a compound selected from the group consisting of wogonin, isoliquiritigenin, coumestrol, and combinations comprising one or more of the foregoing compounds;
oridonin; and
beta-pachyman.

**31.** A composition, comprising:

about 1 to about 30 weight percent of wogonin, isoliquiritigenin, coumestrol, or a combination comprising one or more of the foregoing compounds;
about 0.1 to about 5 weight percent of oridonin; and
about 20 to about 90 weight percent of beta-pachyman;

wherein all weight percents are based on the total weight of the composition.

**32.** A method of treating a human in need of treatment for an estrogen- related disorder, comprising administering a composition comprising greater than 0.5 weight percent based on the total weight of the composition of wogonin, its pharmaceutically acceptable salts and esters, its selectively substituted analogs, or a combination of one or more of the foregoing compounds.

**33.** The method of Claim 32, wherein the estrogen-related disorder is selected from the group consisting of bone loss, bone fractures, osteoporosis, glucocorticoid induced osteoporosis, Paget's disease, abnormally increased bone turnover, periodontal disease, tooth loss, rheumatoid arthritis, osteoarthritis, periprosthetic osteolysis, osteogenesis imperfecta, metastatic bone disease, hypercalcemia of malignancy, cartilage degeneration, endometriosis, uterine fibroid disease, hot flashes, cardiovascular disease, impairment of cognitive function, cerebral degenerative disorders, restenosis, gynecomastia, vascular smooth muscle cell proliferation, obesity, incontinence, the symptoms of menopause, and combinations comprising one or more of the foregoing disorders.

**34.** The method of Claim 32, wherein the woginin comprises a glycoside of wogonin; a selectively substituted analog of wogonin selected from the group consisting of genistein, biochanin, prunetin, scutellarein, daidzin, luteolin, apigenin, acacetin, 3,6, 4-trihydoxylflavone, 7,3-dihydroxy-4, 1-dimethoxy-isoflavone, 3R-2', 3'- dihydoxy-7, 4-dimethoxy-isoflavone; or a combination of one or more of the foregoing compounds.

**35.** The method of Claim 32, wherein treating comprises administering a dosage of about 0.01 mg/kg/day to about 600 mg/kg/day of wogonin.

**36.** The method of Claim 32, wherein the composition further comprises isoliquiritigenin, coumestrol, or a combination of one or more of the foregoing compounds.

FIG. 1

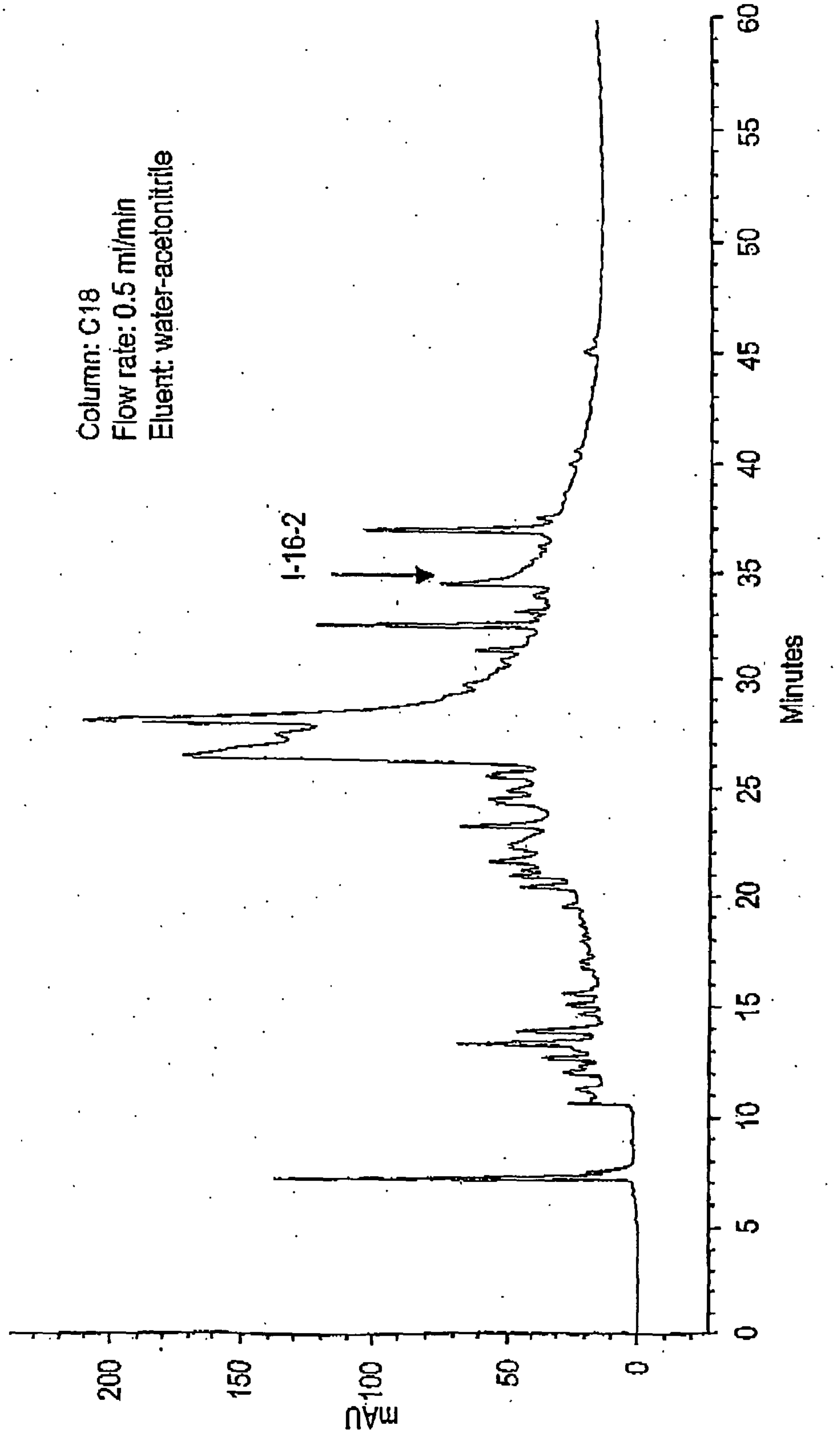
Column: C18
Flow rate: 0.5 ml/min
Eluent: water-acetonitrile
I-16-2
mAU
200
150
100
50
0
Minutes
0
5
10
15
20
25
30
35
40
45
50
55
60

FIG. 2A

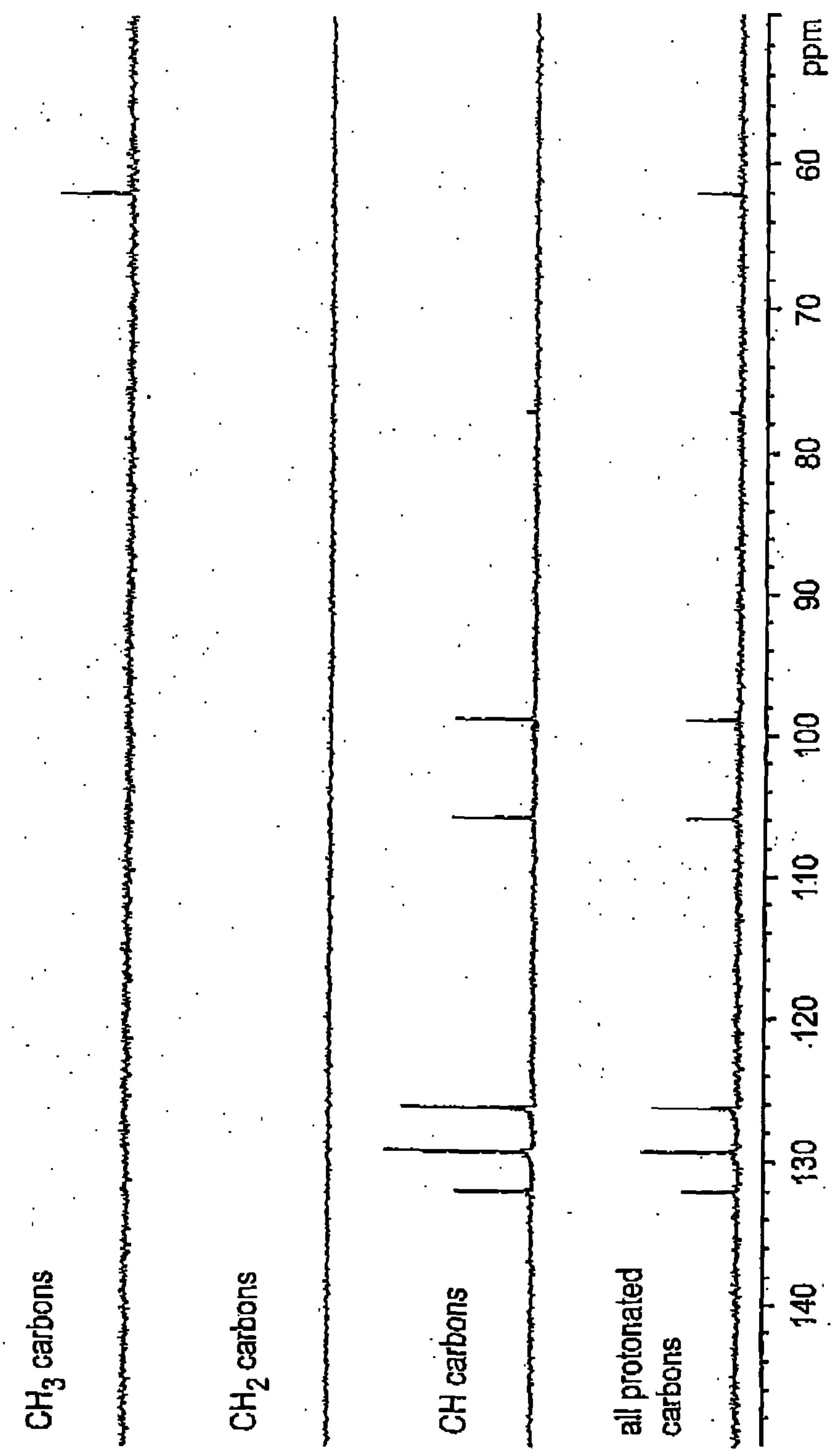
CH3 carbons
CH2 carbons
CH carbons
all protonated carbons
ppm
60
70
80
90
100
110
120
130
140

FIG. 2B

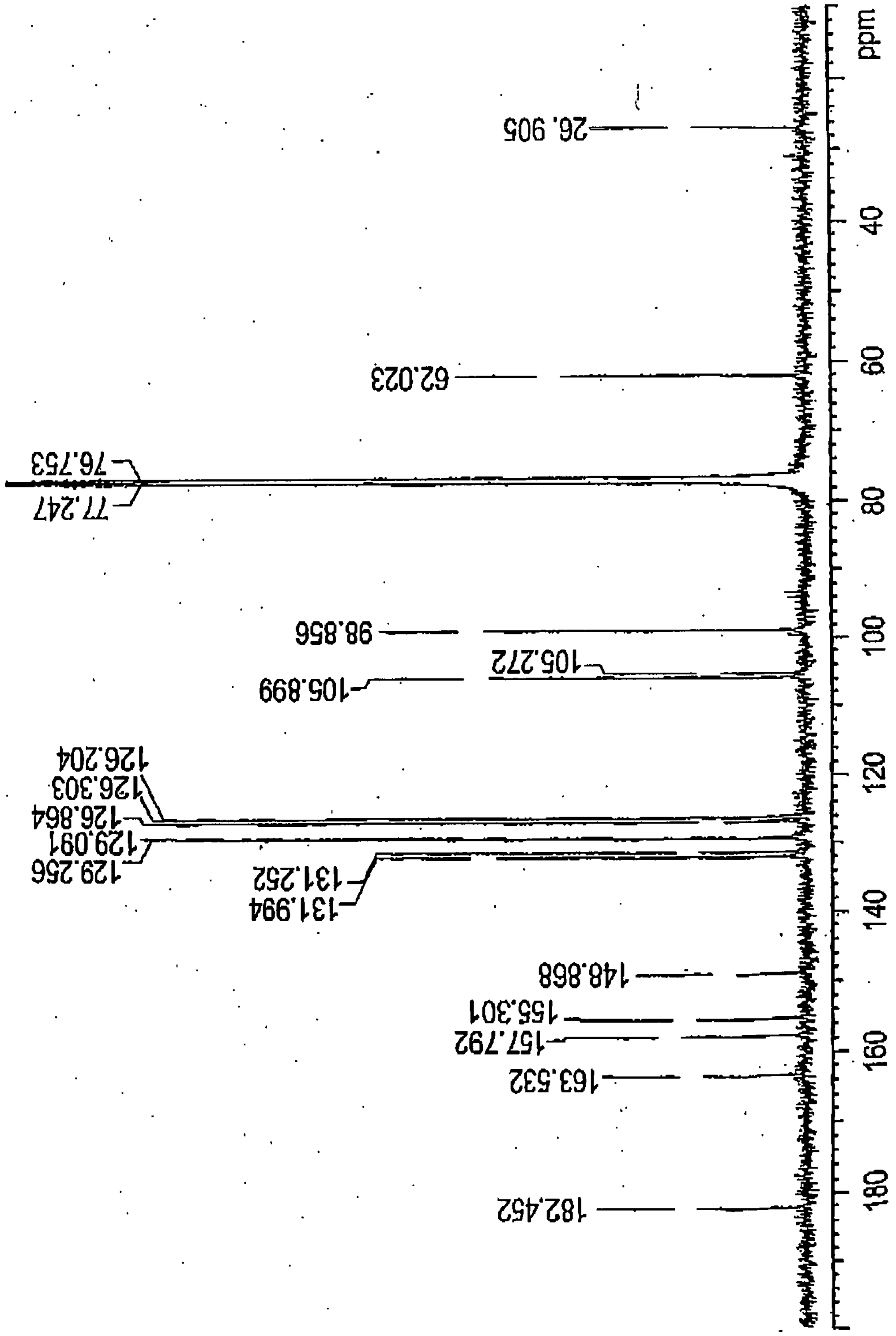

ppm
26.905
62.023
76.753
77.247
98.856
105.272
105.899
126.204
126.303
126.864
129.091
129.256
131.252
131.994
148.868
155.301
157.792
163.532
182.452
40
60
80
100
120
140
160
180

FIG. 3

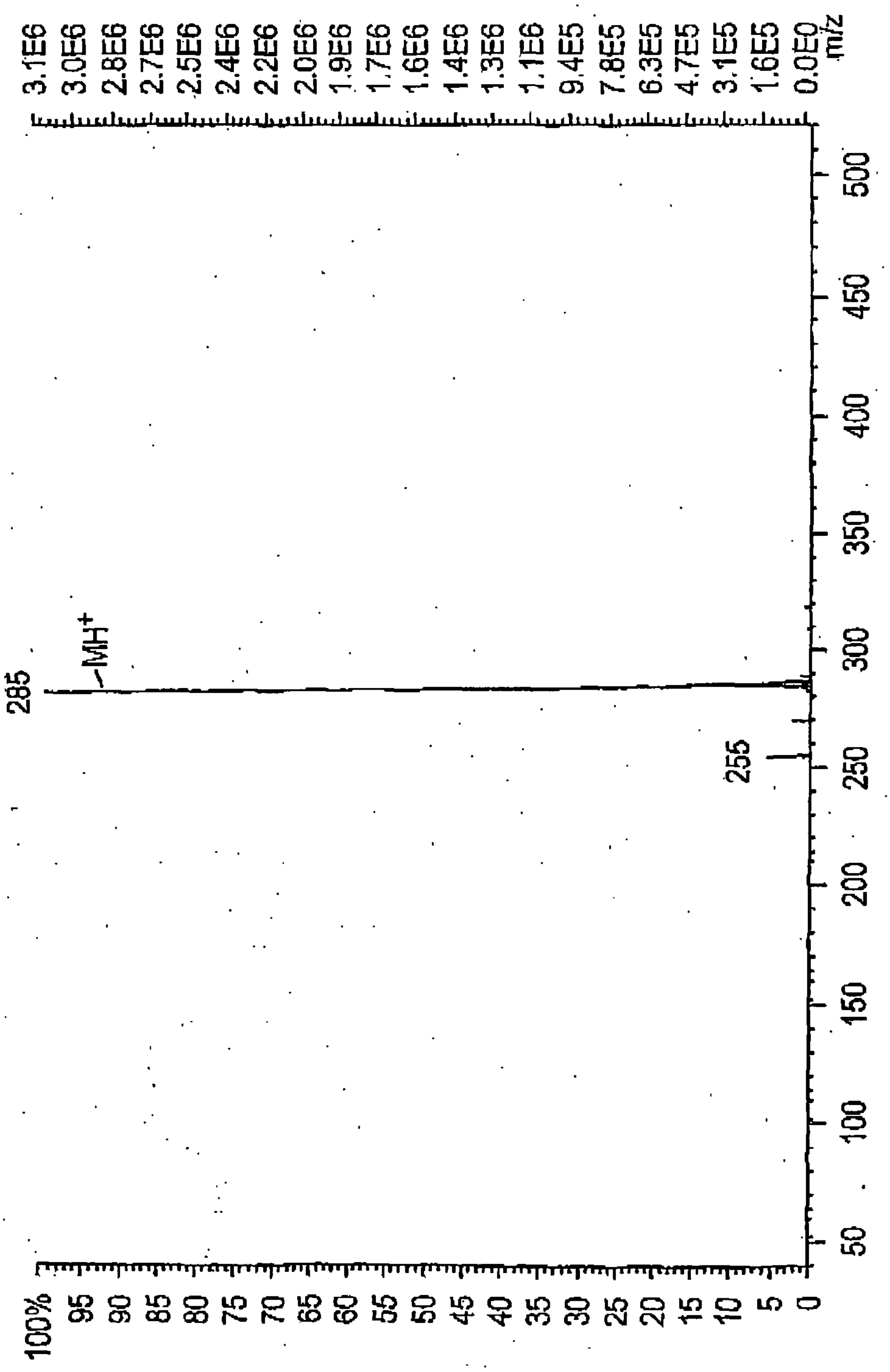
285
MH+
255
100%
95
90
85
80
75
70
65
60
55
50
45
40
35
30
25
20
15
10
5
0
3.1E6
3.0E6
2.8E6
2.7E6
2.5E6
2.4E6
2.2E6
2.0E6
1.9E6
1.7E6
1.6E6
1.4E6
1.3E6
1.1E6
9.4E5
7.8E5
6.3E5
4.7E5
3.1E5
1.6E5
0.0E0
50
100
150
200
250
300
350
400
450
500
m/z

FIG. 4

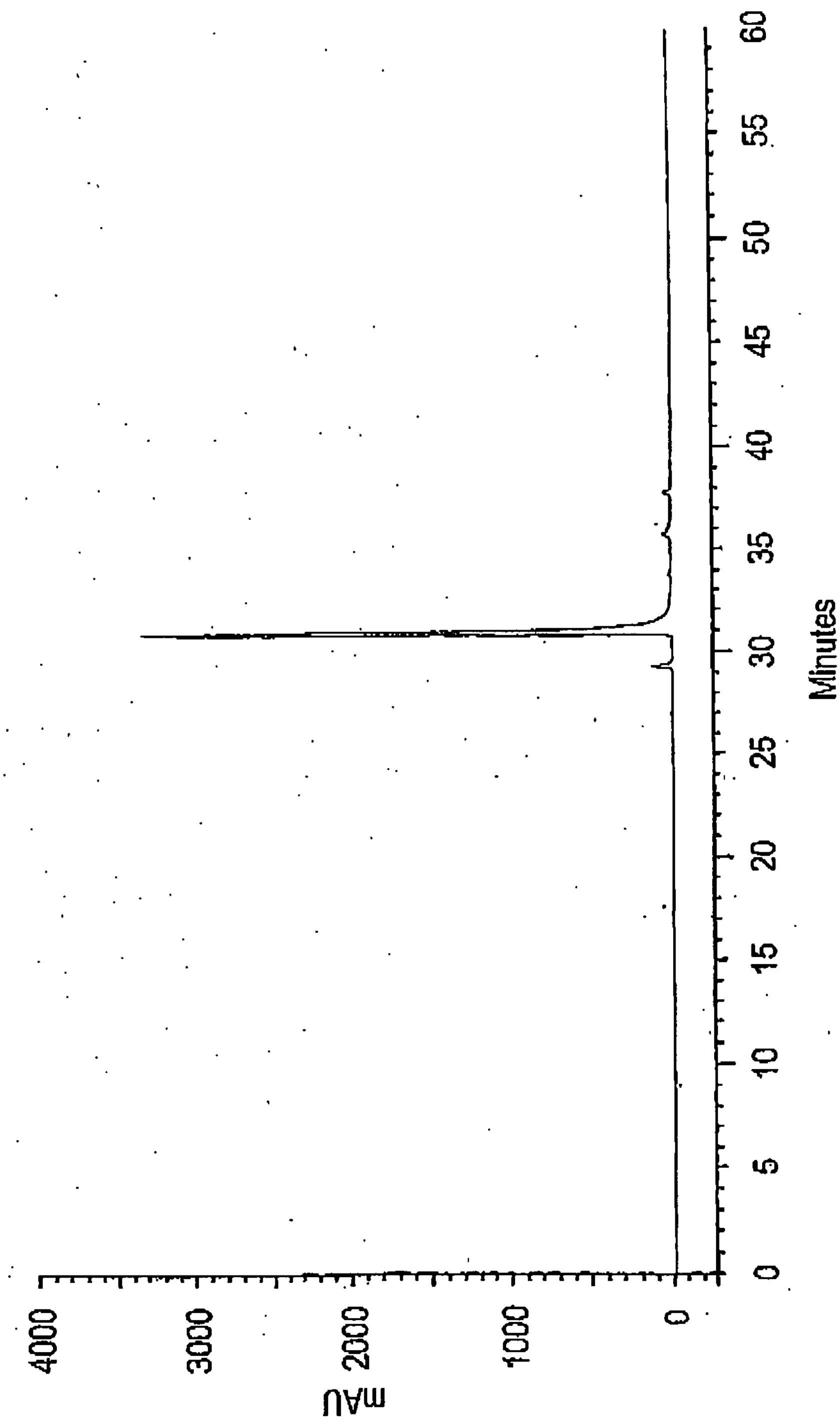
mAU
4000
3000
2000
1000
0
Minutes
0
5
10
15
20
25
30
35
40
45
50
55
60

FIG. 5

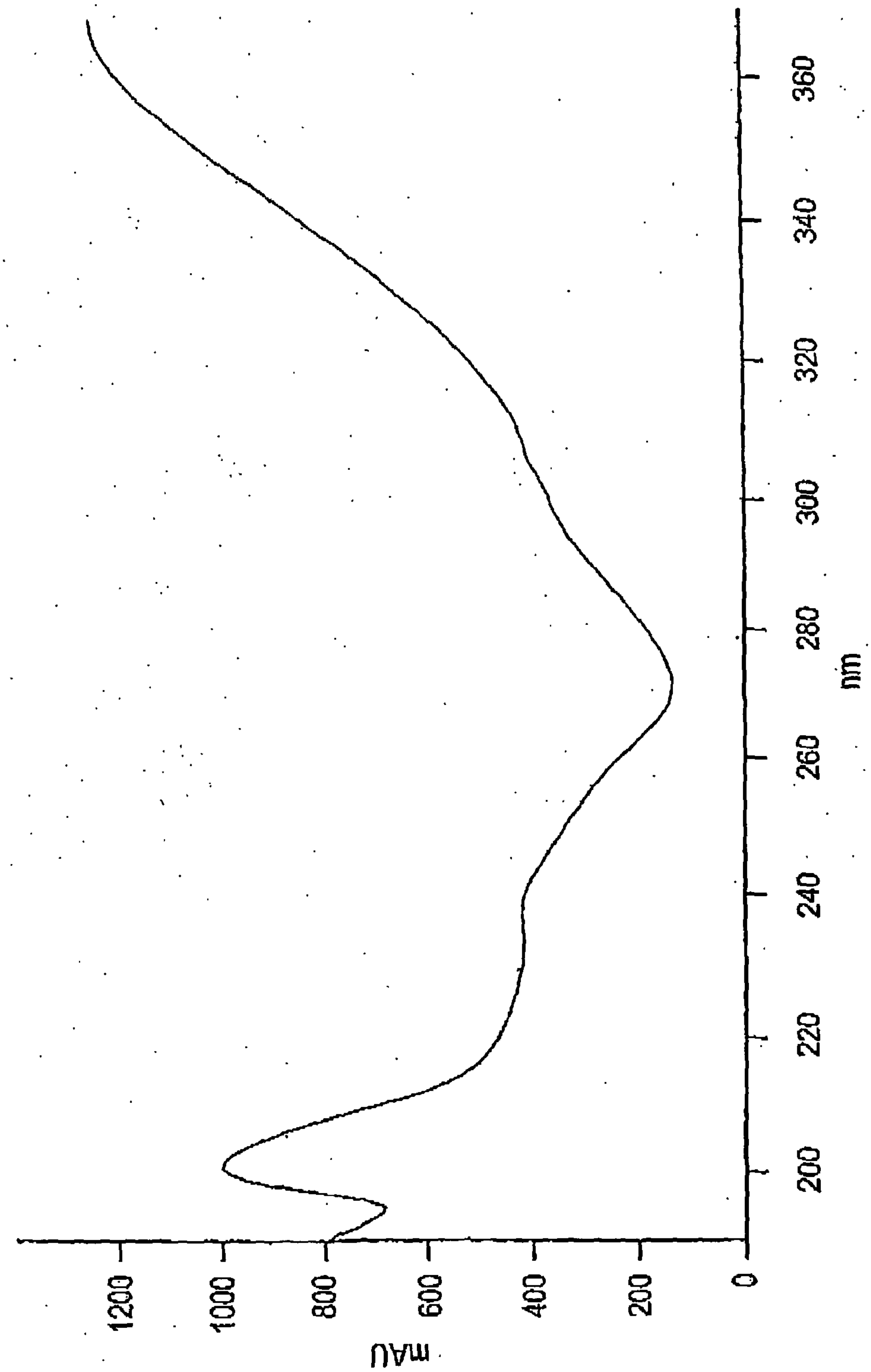
mAU
0
200
400
600
800
1000
1200
nm
200
220
240
260
280
300
320
340
360

FIG. 6A

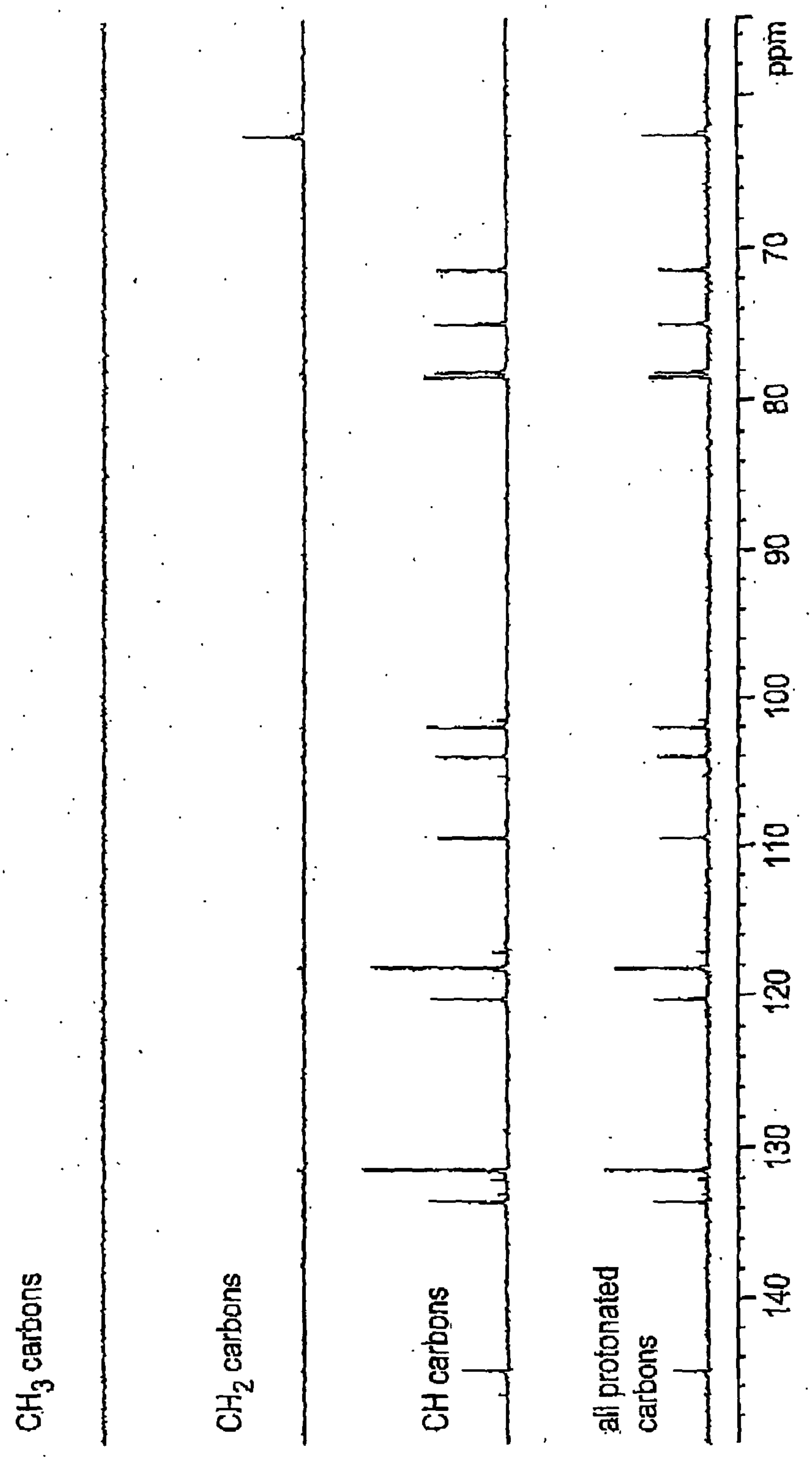
CH3 carbons
CH2 carbons
CH carbons
all protonated carbons
ppm
70
80
90
100
110
120
130
140

FIG. 6B

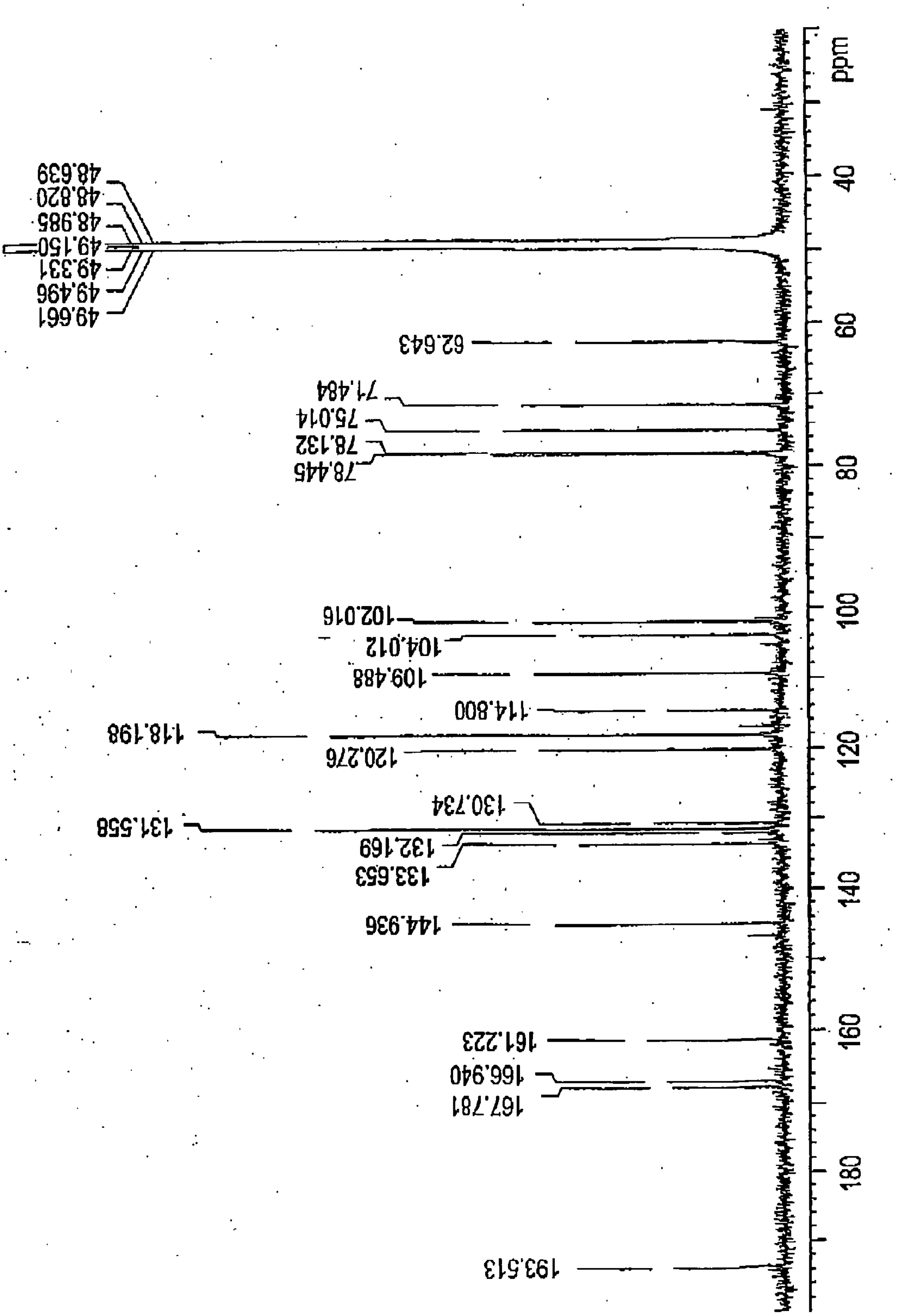
48.639
48.820
48.985
49.150
49.331
49.496
49.661
62.643
71.484
75.014
78.132
78.445
102.016
104.012
109.488
114.800
118.198
120.276
130.734
131.558
132.169
133.653
144.936
161.223
166.940
167.781
193.513
ppm
40
60
80
100
120
140
160
180

FIG. 7

Isoliquirtigenin

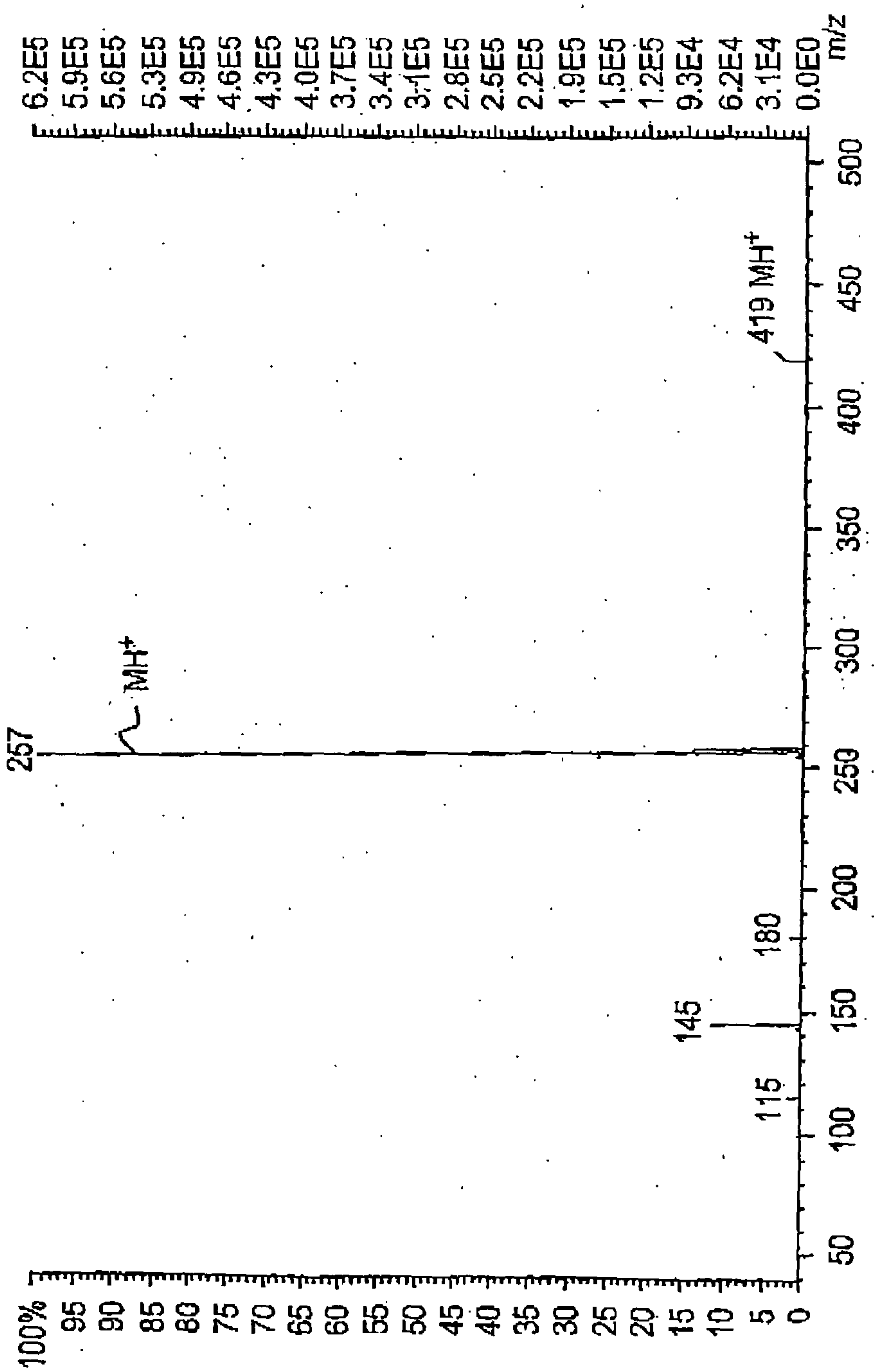

FIG. 8

Cell viability (MTT) curves for Wogonin treatment of LNCaPand DU145

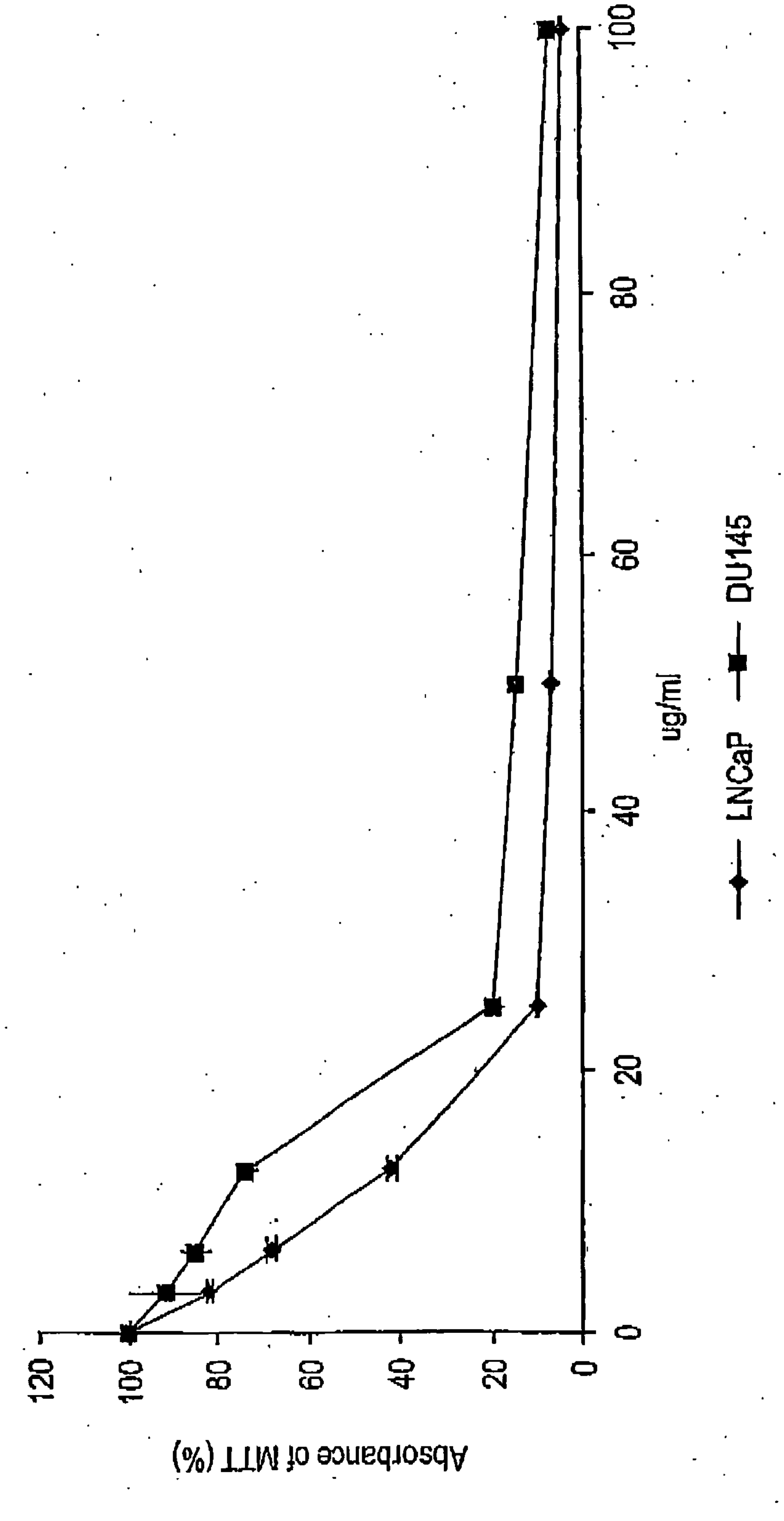

FIG. 9

Cell viability (MTT) curves for Isoliquirtigenin treatment of LNCaP, DU145 and MCF-7

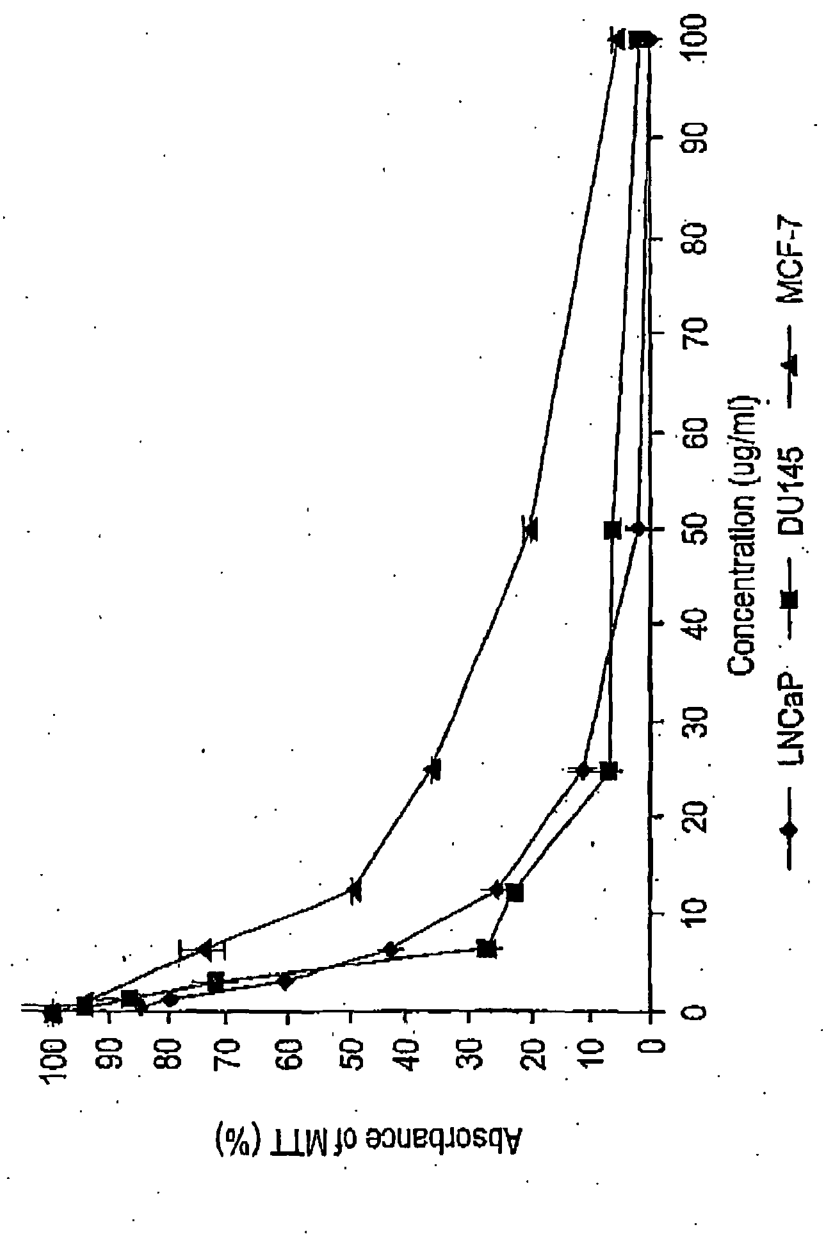

FIG. 10A

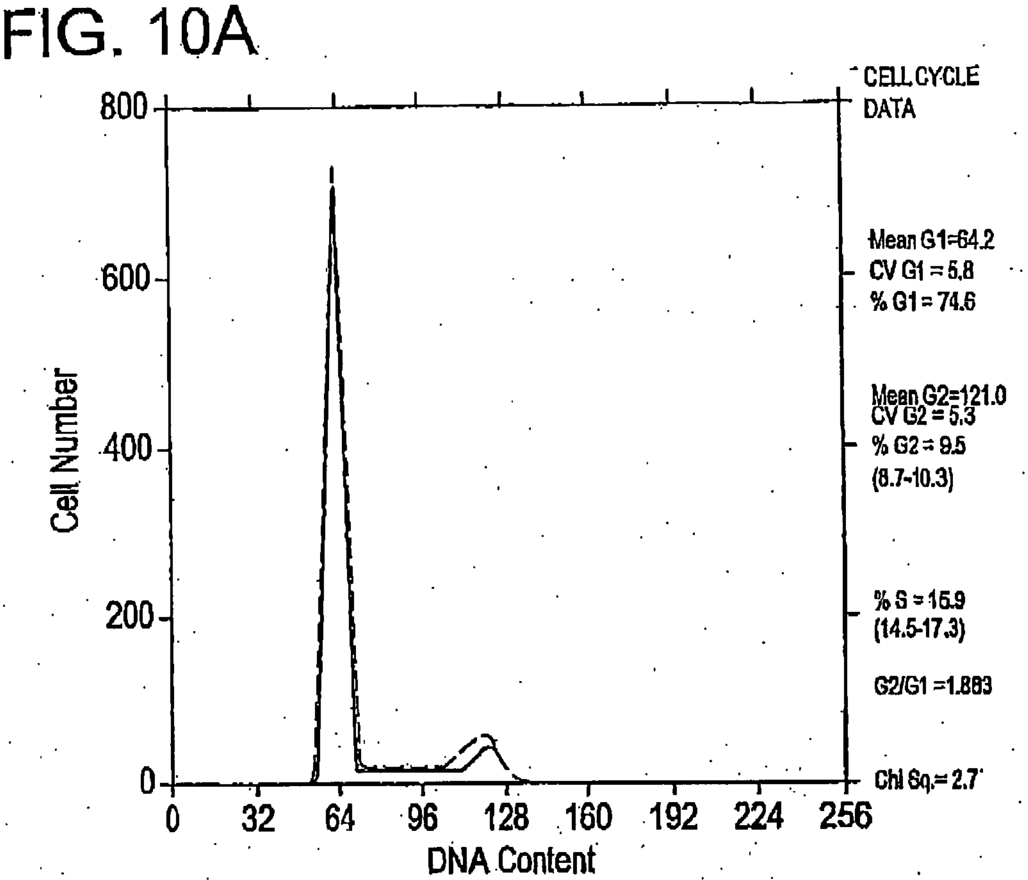


FIG. 10B

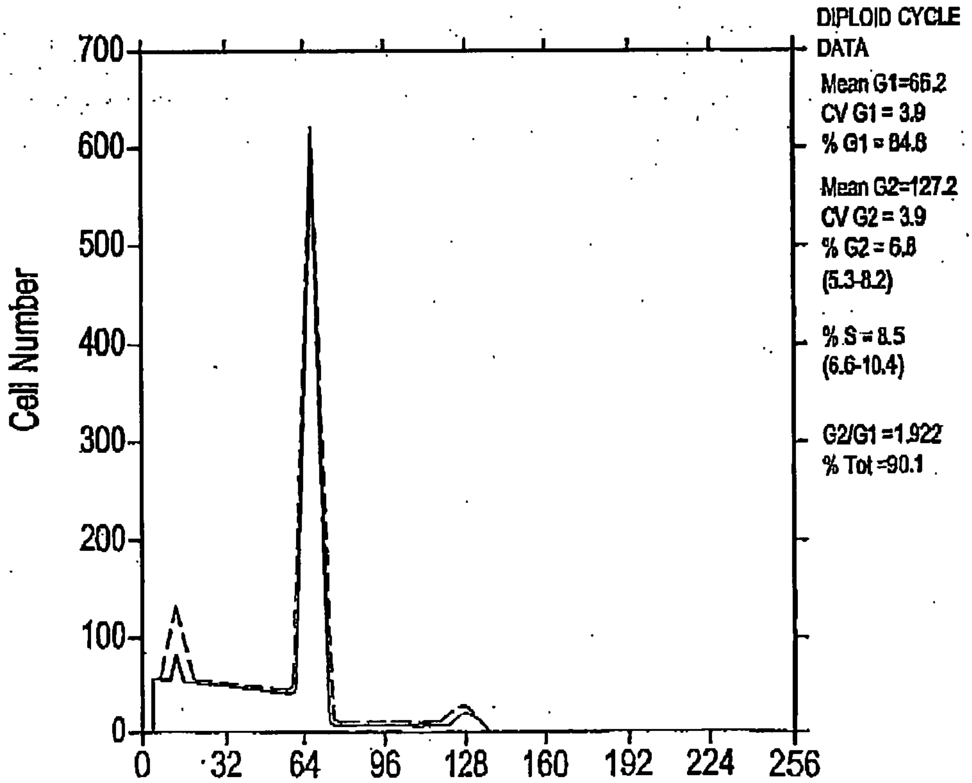

FIG. 11

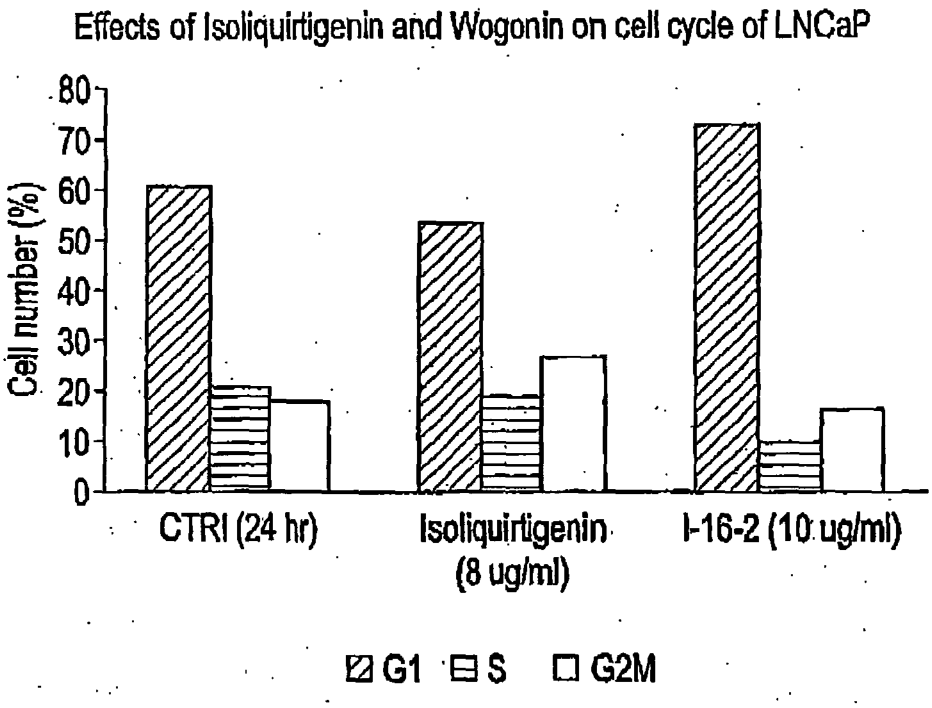
Effects of Isoliquirtigenin and Wogonin on cell cycle of LNCaP
Cell number (%)
80
70
60
50
40
30
20
10
0
CTRl (24 hr)
Isoliquirtigenin (8 ug/ml)
I-16-2 (10 ug/ml)
G1
S
G2M

FIG. 12

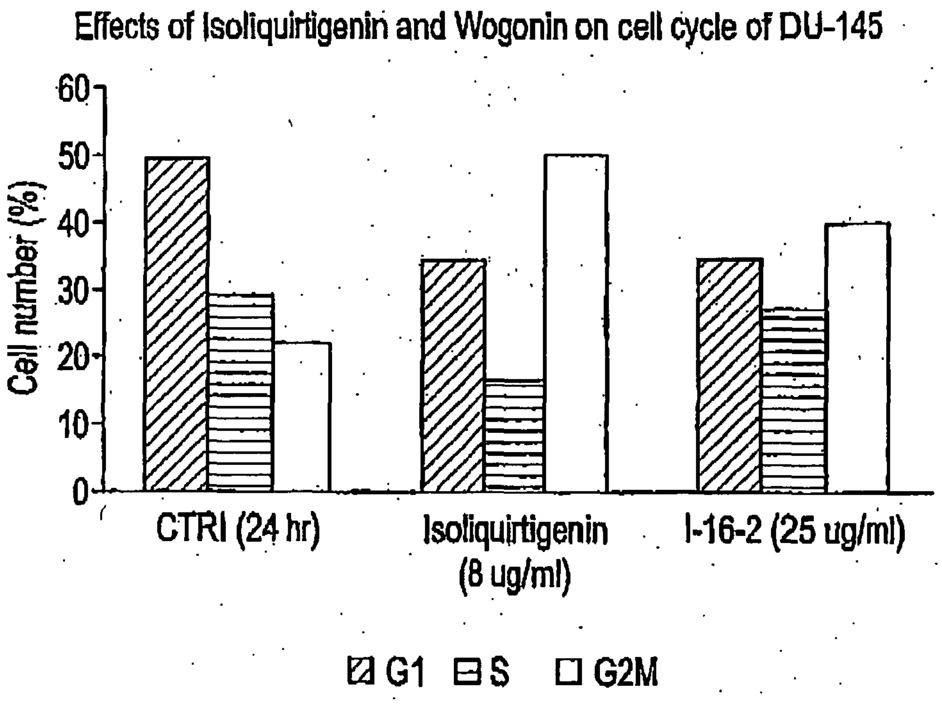
Effects of Isoliquirtigenin and Wogonin on cell cycle of DU-145
Cell number (%)
60
50
40
30
20
10
0
CTRl (24 hr)
Isoliquirtigenin (8 ug/ml)
I-16-2 (25 ug/ml)
G1
S
G2M

FIG. 13

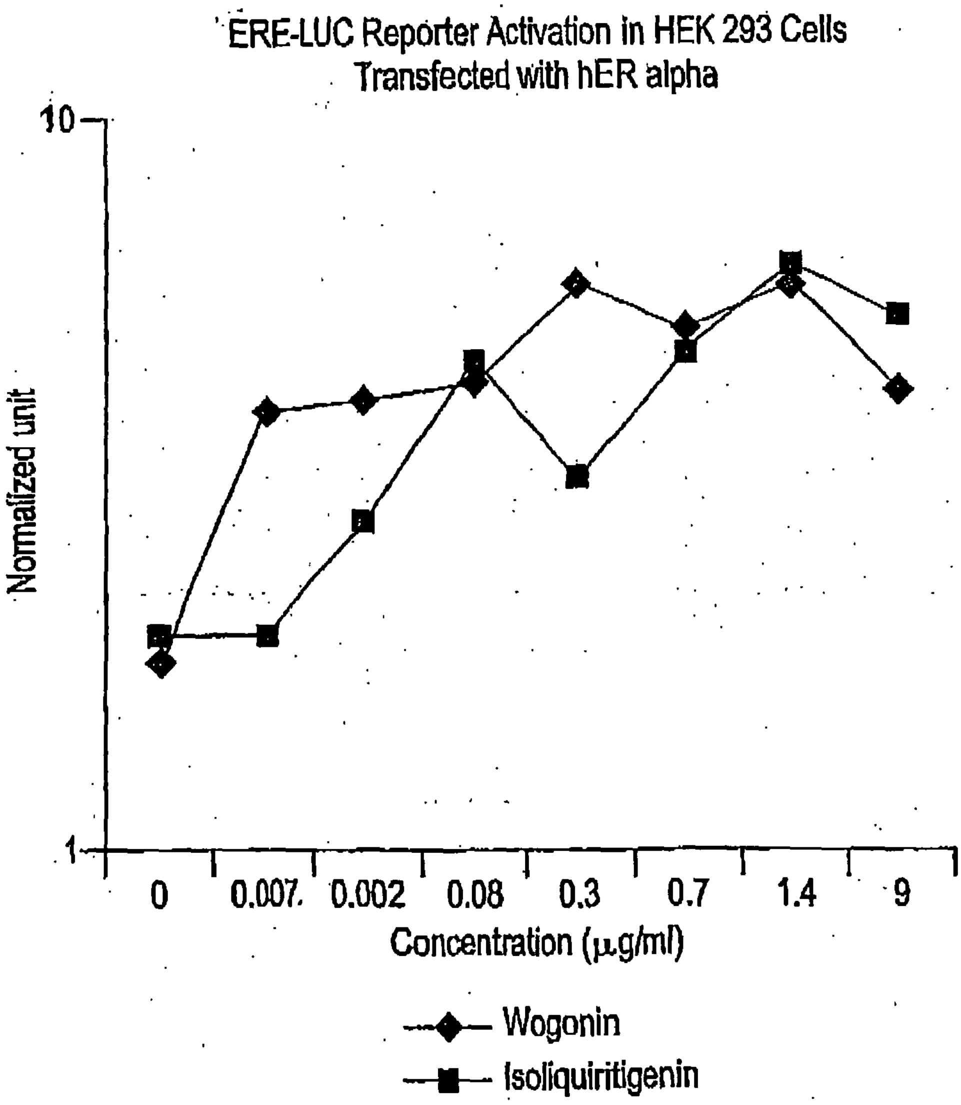
ERE-LUC Reporter Activation in HEK 293 Cells
Transfected with hER alpha
10
1
Normalized unit
0
0.007
0.002
0.08
0.3
0.7
1.4
9
Concentration (μg/ml)
Wogonin
Isoliquiritigenin

FIG. 14

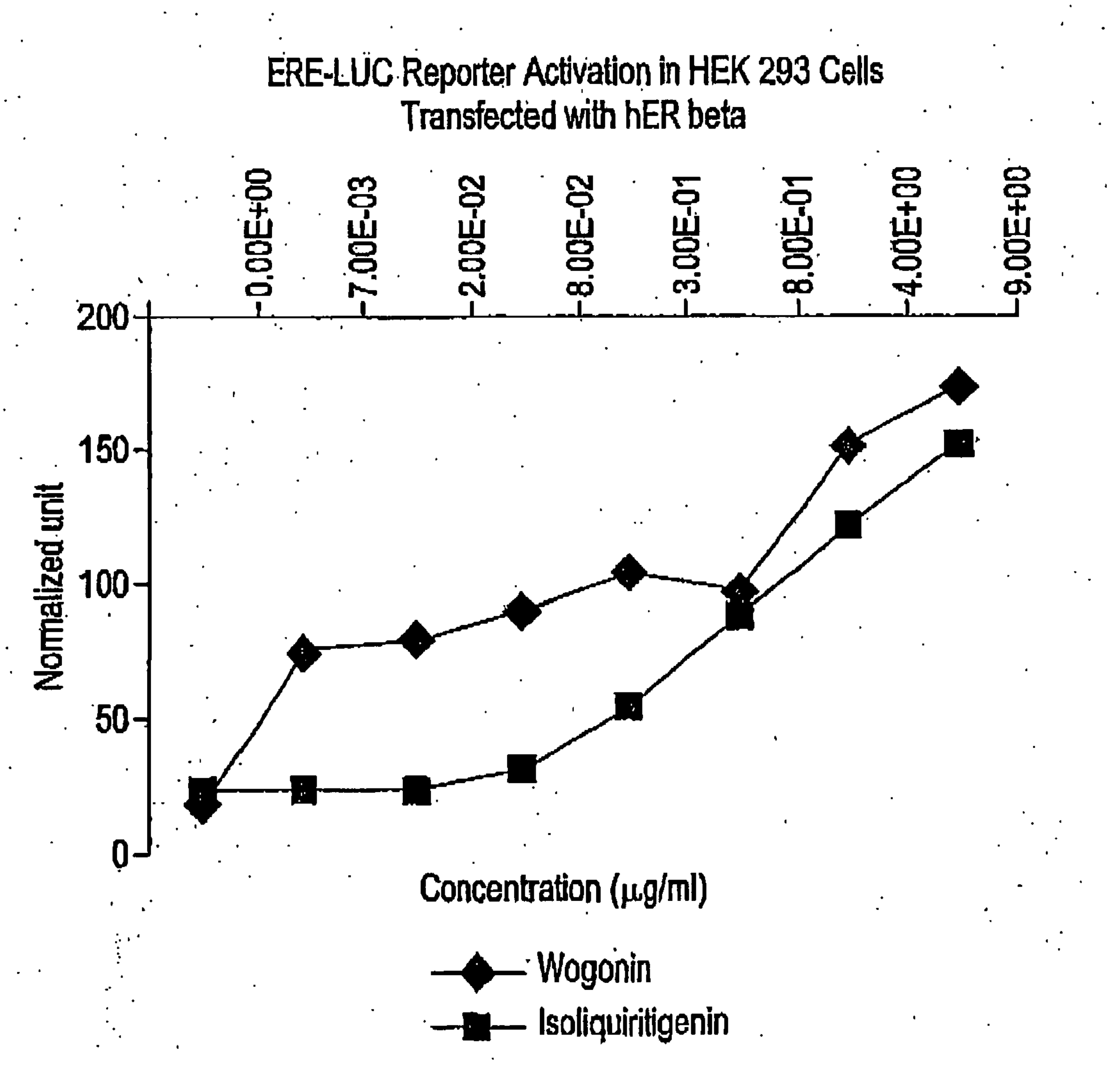

ERE-LUC Reporter Activation in HEK 293 Cells
Transfected with hER beta
0.00E+00
7.00E-03
2.00E-02
8.00E-02
3.00E-01
8.00E-01
4.00E+00
9.00E+00
200
150
100
50
0
Normalized unit
Concentration (µg/ml)
Wogonin
Isoliquiritigenin

FIG. 15

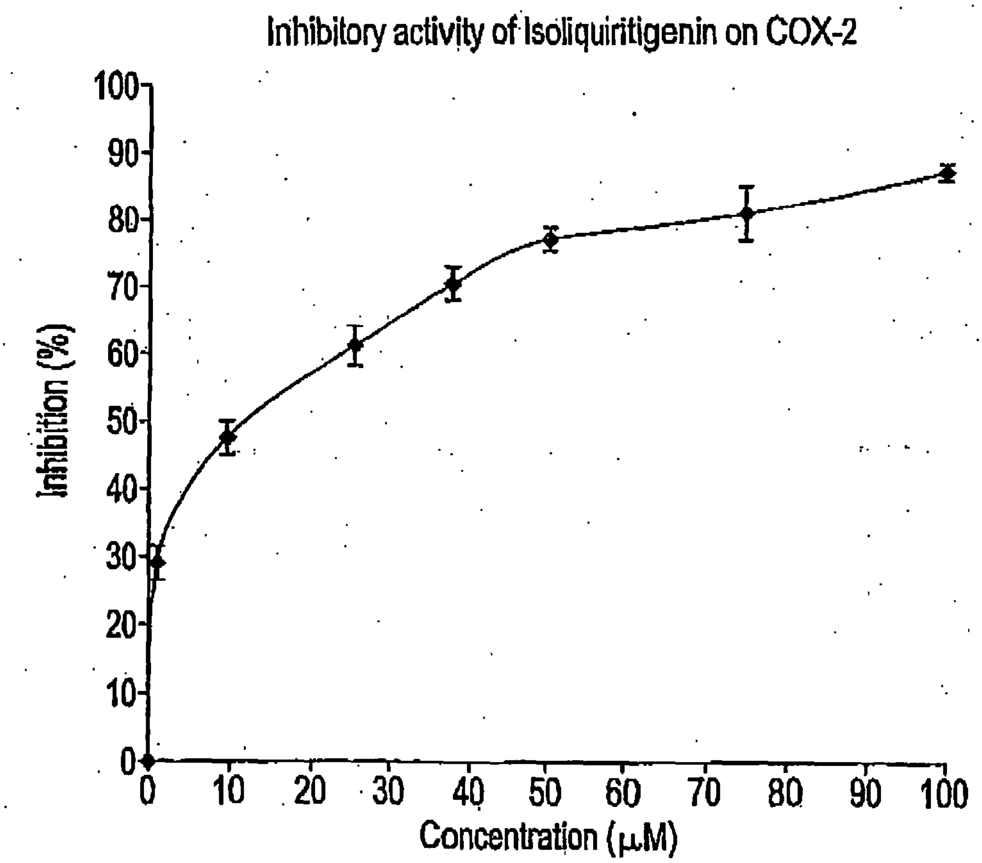
Inhibitory activity of Isoliquiritigenin on COX-2
100
90
80
70
60
50
40
30
20
10
0
Inhibition (%)
0
10
20
30
40
50
60
70
80
90
100
Concentration (μM)

FIG. 16

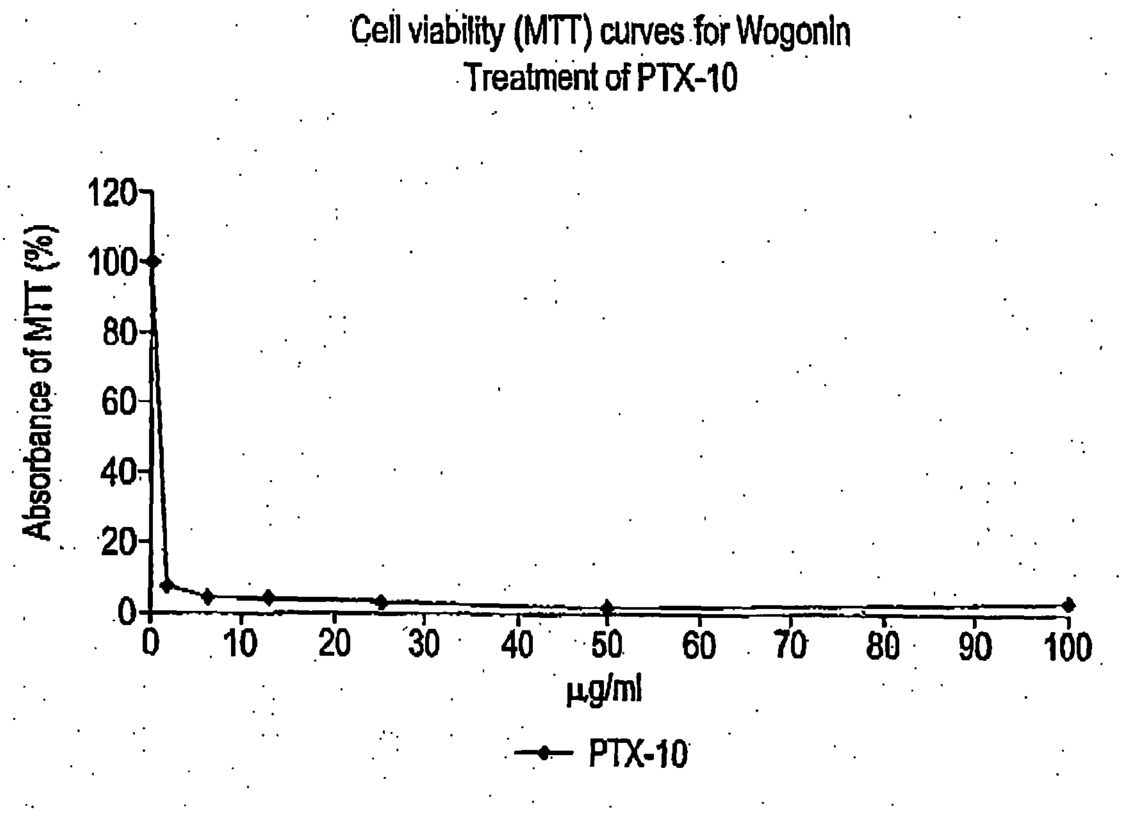
Cell viability (MTT) curves for Wogonin
Treatment of PTX-10
Absorbance of MTT (%)
120
100
80
60
40
20
0
0
10
20
30
40
50
60
70
80
90
100
µg/ml
PTX-10

FIG. 17

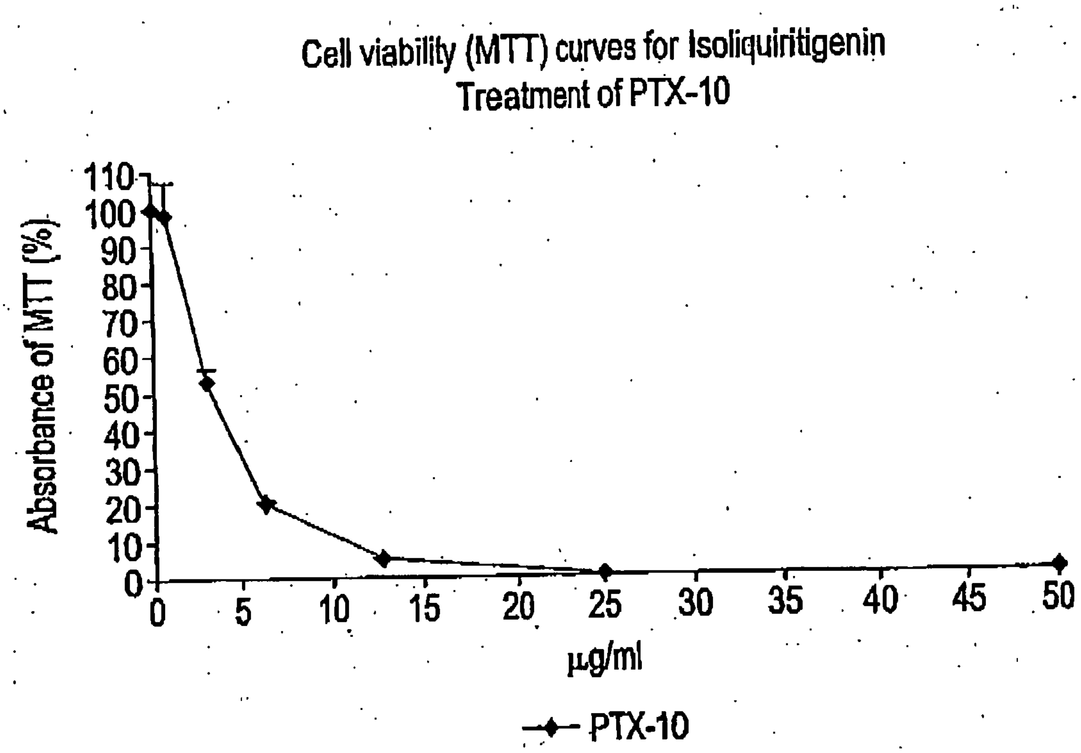
Cell viability (MTT) curves for Isoliquiritigenin
Treatment of PTX-10
Absorbance of MTT (%)
110
100
90
80
70
60
50
40
30
20
10
0
0
5
10
15
20
25
30
35
40
45
50
µg/ml
PTX-10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20010044431 A1, Rodriguez
- WO 01051482 A1, Wallace
- US 3472931 A, Stoughton
- US 3891757 A, Higuchi
- GB 1001949 A, Meyer
- GB 1464975 A, Astra Lakemedel


### Non-patent literature cited in the description


- **HENDERSON et al.** Hormonal carcinogenesis. *Carcinogenesis,* 2000, vol. 21 (3), 427-433
- **H. WISEMAN.** The therapeutic potential of phytoestragens. *Expert. Opin. Investig. Drugs,* 2000, vol. 9 (8), 1829-40
- **S. H. LANDIS et al.** Cancer Statistics. *CA Cancer J. Clin.,* 1998, vol. 48, 6-29
- *National Alliance of Breast Cancer Organizations News,* January 2001, vol. 15 (1), 2
- **Y. Y. ZHANG et al.** Comparative study of Scutellaria planipes and Scutellaria baicalensis. *Biomed. Chromatogra,* 1998, vol. 12, 31-3
- **H. HAYASHI H. et al.** Seasonal variation of glycyrrhizain and isoliquiritgenin glycosides in the root of glycyrrhiza glabra L. *Biol. Pharm. Bull.,* 1998, vol. 21, 987-9
- **Y. S. CHI et al.** Effect of wogonin, a plant flavone from Scutellaria radix, on the expression of cyclooxygenase-2 and the induction of inducible nitric oxide synthase , and the induction of inducible nitric oxide synthase in inhibitors and lipopolysaccharide-treated RAW 264.7 cells. *Biochem. Pharmacol.,* 2001, vol. 61 (11), 1417-27
- **S. TAMIR.** Estrogen-like activity of glabrene and other constituents isolated from licorice root. *J. Steroid Biochem. Mol. Biol.,* 2001, vol. 78 (3), 291-8
- **A. KIRSCHENBAUM et al.** The role of cyclocxygenase-2 in prostate cancer. *Urology,* 2001, vol. 58 (2), 127-131
- **E. T. HAWK et al.** COX-2 in Cancer-A Player That's Defining the Rules. *J. Natl. Cancer Inst.,* 2002, vol. 94 (8), 545-546
- **E. FOSSLIEN.** Review: Molecular pathology of eyclooxygenas-2 in cancer-induced angiogenesis. *Ann. Clin. Lab Sci.,* 2001, vol. 31 (4), 325-348
- **P. RIVAILLE et al.** *C. R. Acad. Sci., ,Paris, Ser. C,* 1969, vol. 268 (2), 2213-16
- **M. -C. LIN et al.** *J. Chromatogr, A,* 1999, vol. 830 (2), 387-395
- **Y. C. CHEN et al.** *Biochem. Pharmacol.,* 2001, vol. 61 (11), 1417-1427
- **S. K. SRIVASTAVA et al.** *Indian J. Chem., Sect. B,* 1981, vol. 20B (4), 347-8
- **F. A. MACIAS et al.** *Phytochemistry,* 1998, vol. 50 (1), 35-46
- **P. M. DEWICK et al.** *J. Chem. Soc. D,* 1969, vol. 9, 466-7
- **T. KAPPE et al.** *Z Naturforsch.; Teil B,* 1974, vol. 29 (3-4), 292-3
- **R LASCHOBER et al.** *Synthesis,* 1990, vol. 5, 387-8
- **K. HIROYA et al.** *Perkin 1,* 2000, vol. 24, 4339-4346
- **G. A. KRAUS et al.** *Journal of Organic Chemistry,* 2000, vol. 65 (18), 5644-5646
- **M. OKADA et al.** *Planta Med.,* 2000, vol. 66 (6), 572-575
- **H. D. HALICKA ; B. ARDELT ; G. JUAN ; A. MITTELMAN ; S. CHEN ; F. TRAGANOS ; Z. DARZYNKIEWICZ.** Apoptosis and Cell Cycle Effects Induced by Extracts of the Chinese Herbal Preparation of PC SPES. *International J. of Oncology,* 1997, vol. 11, 437-448
- **YOON et al.** Differential activation of wild-type and variant forms of estrogen receptor $\alpha$ by synthetic and natural estrogenic compounds using a promoter containing three estrogen-responsive elements. *J. Steroid Biochem. & Molecular Biology,* 2000, vol. 28, 25-32
- **TZUKERMAN et al.** Human estrogen receptor transactivational capacity is determined by both cellular and promoter content and mediated by two functionally distinct intermolecular regions. *Mol. Endocrinol.,* 1994, vol. 8, 21-30
- **W. XIE ; J. G. CHIPMAN ; D. L, ROBERTSON et al.** Expression of a mitogen-responsive gene encoding prostaglandin synthase is regulated by mRNA splicing. *Proc. Natl. Acad. Sci,* 1991, vol. 88, 2692-2696
- **K.M. MAXEY ; K.R. MADDIPATI ; J. BIRKMEIER.** Interference in enzyme immunoassays. *J. Clin. Immunoassay,* 1992, vol. 15, 116-120

- **P. PRADELLES ; J. GRASSI ; J. A. MACLOUF.** Enzyme immunoassays of eicosanoids using acetylcholinesterase as label: An alternative to radioimmunoassay. *Anal. Chem.,* 1985, vol. 57, 1170-1173
- **MACLOUF J. ; GRASSI, J. ; PRADELLES, P.** Development of enzyme-immunoassay techniques for the measurement of eicosanoids. *Prostaglandin and Lipid Metabolism in Radiation Injury,* 1987, 355-364